# EUROPEAN PATENT APPLICATION

(11) **EP 4 190 893 A1**
(43) Date of publication of application: **07.06.2023**
(21) Application number: 21850475.1
(22) Date of filing: 30.07.2021
(51) Int. Cl.: C12N 5/071, C12Q 1/02, A61L 27/36, A61L 27/38, A61L 27/40, A01K 67/027

(54) **BLADDER ORGANOID AND METHOD FOR PRODUCING SAME**

(30) Priority: 30.07.2020 JP 2020129498
(71) Applicant: RIKEN, Wako-shi, Saitama 351-0198 (JP); Otsuka Pharmaceutical Co., Ltd., Tokyo 101-8535 (JP)
(72) Inventor: TAKASATO, Minoru, Wako-shi, Saitama 351-0198 (JP); OFUJI, Kazuhiro, Wako-shi, Saitama 351-0198 (JP); WYMEERSCH, Filip Jos, Wako-shi, Saitama 351-0198 (JP)
(74) Representative: Hoffmann Eitle
(86) International application number: PCT/JP2021/028394
(87) International publication number: WO 2022/025269

(57) **Abstract**

An object of the present invention is to provide a ventral hindgut organoid for producing a bladder organoid that comprises a layer structure of bladder epithelial cell types like the urinary bladder. An aspect of the present invention is to provide a method for producing a ventral hindgut organoid, comprising culturing a pluripotent stem cell with an inducer medium A containing activin A and GSK3β inhibitor to induce differentiation into definitive endoderm cells and culturing the definitive endoderm cells with an inducer medium B containing fibroblast growth factor, GSK3β inhibitor, and optionally further containing bone morphogenetic protein, and then culturing them in the presence of extracellular matrix with an inducer medium B containing fibroblast growth factor, GSK3β inhibitor, and optionally further containing bone morphogenetic protein to form a ventral hindgut organoid.

## Description

### TECHNICAL FIELD

The present disclosure relates to a ventral hindgut organoid, a bladder organoid, or a method for producing thereof. The present disclosure also relates to a non-human mammal comprising a ventral hindgut organoid or bladder organoid in a kidney or bladder thereof or its peripheral area or a method for producing the same. The present disclosure relates to a method for assessing drug responsiveness to a test substance in a ventral hindgut organoid, a bladder organoid, or a non-human mammal. The present disclosure further relates to a regenerative medicine composition comprising a ventral hindgut organoid or a bladder organoid.

### BACKGROUND ART

The bladder is an organ derived from the embryonic endoderm and is known to originate from the hindgut, located at the most posterior part of the early gut tube, through the ventral cloaca. The bladder is a pouch-like organ temporarily holding urine, which is delivered from the kidney through the ureter and excreted through the urethra. Bladder functions to hold or excrete urine may reduce or lose when bladder tissue is injured by radiotherapy, bladder rupture, diabetes, and the like.

Regenerative medicine research is conducted to aim to regenerate dysfunctional organs, treat intractable diseases, or compensate for the chronic shortage of donors for organ transplantation. In the technical field of regenerative medicine, organ-like cell assemblies, called organoids, generated *in vitro* from pluripotent stem cells such as ES cells or iPS cells are being investigated.

Non-Patent Document 1 describes that bladder epithelium was induced and differentiated by culturing mouse ES cells *in vitro.* Non-Patent Documents 2-4 describe that bladder epithelium was induced and differentiated by culturing human pluripotent stem cells *in vitro.* Non-Patent Document 5 describes that bladder epithelium was induced and differentiated by culturing mouse ES cells in a collagen matrix *in vitro* and then transplanting them under the renal capsule of a mouse.

### CITATION LIST

Non-Patent Document 1: Mauney JR, et. al., All-Trans Retinoic Acid Directs Urothelial Specification of Murine Embryonic Stem Cells via GATA4/6 Signaling Mechanisms. PLoS One 2010, 5:e11513.
Non-Patent Document 2: Osborn SL, et. al., Induction of human embryonic and induced pluripotent stem cells into urothelium. Stem Cells Transl Med 2014, 3:610-9.
Non-Patent Document 3: Suzuki K, et. al., Directed differentiation of human induced pluripotent stem cells into mature stratified bladder urothelium. Sci Rep 2019, 9:1-13.
Non-Patent Document 4: Kang M, et. al., Generation of Bladder Urothelium from Human Pluripotent Stem Cells under Chemically Defined Serum- and Feeder-Free System. Int J Mol Sci 2014, 15:7139-7157.
Non-Patent Document 5: Oottamasathien S, et. al., Directed differentiation of embryonic stem cells into bladder tissue. Dev Biol 2007, 304:556-566.

### SUMMARY

### TECHNICAL PROBLEM

The induction of differentiation into bladder epithelium described in Non-Patent Documents 1-4 neither mimics the actual developmental process of the bladder nor induces the differentiation in a system of three-dimensional culturing. Bladder epithelial organoids induced and differentiated in Non-Patent Documents 1-4 do not have a layer structure of bladder epithelial cell types as in the bladder. Bladder epithelial organoids induced and differentiated in Non-Patent Document 5 are uncertain about having a three-layer structure like the bladder.

The present inventors examined various conditions for forming bladder organoids from pluripotent stem cells. For example, the inventors examined conditions for constructing a three-dimensional culture system that mimics the actual developmental process of the bladder. One object of the present disclosure is to provide a new ventral hindgut organoid for producing a bladder organoid with a layer structure of bladder epithelial cell types like the bladder. One object of the present disclosure is to provide a novel bladder organoid with a layer structure of bladder epithelial cell types like the bladder or a method for producing the same. One object of the present disclosure is to provide a novel non-human mammal comprising a ventral hindgut organoid or bladder organoid in a kidney or bladder thereof or its peripheral area or a method for producing the same. One object of the present disclosure is to provide a novel regenerative medicine composition that comprises a ventral hindgut organoid or bladder organoid. One object of the present disclosure is to provide a novel method for assessing drug responsiveness to a test substance in a ventral hindgut organoid, bladder organoid, or non-human mammal.

### SOLUTION TO PROBLEM

The present disclosure is to provide the following aspects:
[Item A1] A method for producing a ventral hindgut organoid, comprising culturing pluripotent stem cells with an inducer medium A containing activin A and GSK3β inhibitor to induce differentiation into definitive endoderm cells;
   culturing the definitive endoderm cells with an inducer medium B containing fibroblast growth factor and GSK3β inhibitor, and then culturing them in an extracellular matrix gel with an inducer medium B containing fibroblast growth factor and GSK3β inhibitor to induce differentiation into a hindgut organoid; and
   culturing the hindgut organoid in an extracellular matrix gel with an inducer medium b2 containing fibroblast growth factor, GSK3β inhibitor, and bone morphogenetic protein to form a ventral hindgut organoid.
[Item A2] A ventral hindgut organoid expressing P63, CDX2, HOXA13, and KRT8 but not expressing SOX2.
[Item A2-1] A ventral hindgut organoid expressing P63, CDX2, HOXA13, and KRT8, but not substantially expressing SOX2.
[Item A3] The ventral hindgut organoid according to Item A2, wherein the expression level of CDX2 in the ventral hindgut organoid is less than the expression level of CDX2 in a hindgut organoid, or CDX2 is not expressed in the ventral hindgut organoid.
[Item A3-1] The ventral hindgut organoid according to Item A2, wherein the expression level of CDX2 in the ventral hindgut organoid is less than the expression level of CDX2 in a hindgut organoid, or CDX2 is not substantially expressed in the ventral hindgut organoid.
[Item A4] A method for producing a bladder organoid, comprising culturing a ventral hindgut organoid produced by the method according to Item A1 or a ventral hindgut organoid according to Item A2 or Item A3 in an extracellular matrix gel with an inducer medium C containing retinoic acid, fibroblast growth factor, and bone morphogenetic protein.
[Item A5] A bladder organoid, comprising a first cell layer localized along the outermost periphery of the bladder organoid, the first cell layer comprising cells co-expressing P63 and KRT5; a second cell layer localized in an inward direction relative to the first cell layer, the second cell layer comprising cells co-expressing P63 and UPK2; and a third cell layer localized in an inward direction relative to the second cell layer, the third cell layer comprising cells expressing UPK2 but not expressing P63.
[Item A5-1] A bladder organoid, comprising a first cell layer localized along the outermost periphery of the bladder organoid, the first cell layer comprising cells co-expressing P63 and KRT5; a second cell layer localized in an inward direction relative to the first cell layer, the second cell layer comprising cells co-expressing P63 and UPK2; and a third cell layer localized in an inward direction relative to the second cell layer, the third cell layer comprising cells expressing UPK2 but not substantially expressing P63.
[Item A6] A method for producing a bladder organoid, comprising introducing a ventral hindgut organoid produced by the method according to Item A1 or a ventral hindgut organoid according to Item A2 or Item A3 into a kidney or bladder or its peripheral area of a human or a non-human mammal.
[Item A7] A bladder organoid having a lumen, comprising a first cell layer localized along the outermost periphery of the bladder organoid, the first cell layer comprising cells co-expressing P63 and KRT5; a second cell layer localized in an inward direction relative to the first cell layer, the second cell layer comprising cells co-expressing P63 and UPK2; and a third cell layer localized in an inward direction relative to the second cell layer and facing the lumen, the third cell layer comprising cells expressing UPK2 but not expressing P63.
[Item A7-1] A bladder organoid having a lumen, comprising a first cell layer localized along the outermost periphery of the bladder organoid, the first cell layer comprising cells co-expressing P63 and KRT5; a second cell layer localized in an inward direction relative to the first cell layer, the second cell layer comprising cells co-expressing P63 and UPK2; and a third cell layer localized in an inward direction relative to the second cell layer and facing the lumen, the third cell layer comprising cells expressing UPK2 but not substantially expressing P63.
[Item A8] A method for producing a non-human mammal comprising a bladder organoid in a kidney or bladder thereof or its peripheral area, the method comprising introducing a ventral hindgut organoid produced by the method according to Item A1, a ventral hindgut organoid according to Item A2, Item A2-1, Item A3, or Item A3-1, a bladder organoid produced by the method according to Item A4 or Item A6, or a bladder organoid according to Item A5, Item A5-1, Item A7, or Item A7-1 into the kidney or bladder or its peripheral area of a non-human mammal.
[Item A9] A non-human mammal comprising a ventral hindgut organoid produced by the method according to Item A1, a ventral hindgut organoid according to Item A2, Item A2-1, Item A3, or Item A3-1, a bladder organoid produced by the method according to Item A4 or Item A6, or a bladder organoid according to Item A5, Item A5-1, Item A7, or Item A7-1 in a kidney or bladder thereof or its peripheral area.
[Item A10] A regenerative medicine composition for treating bladder injury or disease, comprising a ventral hindgut organoid produced by the method according to Item A1, a ventral hindgut organoid according to Item A2, Item A2-1, Item A3, or Item A3-1, a bladder organoid produced by the method according to Item A4 or Item A6, or a bladder organoid according to Item A5, Item A5-1, Item A7, or Item A7-1
[Item A11] A method for assessing drug responsiveness to a test substance, compri sing
   contacting the test substance with a ventral hindgut organoid produced by the method according to Item A1, a ventral hindgut organoid according to Item A2, Item A2-1, Item A3, or Item A3-1, a bladder organoid produced by the method according to Item A4 or Item A6, a bladder organoid according to Item A5, Item A5-1, Item A7, or Item A7-1, or a non-human mammal according to Item A9; and
   measuring drug responsiveness to the test substance in the ventral hindgut organoid, the bladder organoid, or the non-human mammal.
[Item A12] A method for treating a bladder injury or disease, comprising introducing a ventral hindgut organoid produced by the method according to Item A1, a ventral hindgut organoid according to Item A2, Item A2-1, Item A3, or Item A3-1, a bladder organoid produced by the method according to Item A4 or Item A6, a bladder organoid according to Item A5, Item A5-1, Item A7, or
Item A7-1 into a kidney or bladder or its peripheral area of a mammal in need thereof.
[Item B1] A method for producing a ventral hindgut organoid, comprising
   culturing a pluripotent stem cell with an inducer medium A containing activin A and GSK3β inhibitor to induce differentiation into definitive endoderm cells and
   culturing the definitive endoderm cells with an inducer medium B containing fibroblast growth factor, GSK3β inhibitor, and optionally further containing bone morphogenetic protein and then culturing them in the presence of extracellular matrix with an inducer medium B containing fibroblast growth factor, GSK3β inhibitor, and optionally further containing bone morphogenetic protein, to form a ventral hindgut organoid.
[Item B2] The method according to Item B1, wherein
   the forming of the ventral hindgut organoid comprises, after culturing the definitive endoderm cells with the inducer medium B, culturing them in the presence of extracellular matrix with the inducer medium B to form a hindgut organoid and culturing the hindgut organoid in the presence of extracellular matrix with the inducer medium B to form the ventral hindgut organoid,
   the inducer medium B for forming hindgut organoid contains the fibroblast growth factor and the GSK3β inhibitor, and the inducer medium B for forming ventral hindgut organoid contains the fibroblast growth factor, the GSK3β inhibitor, and the bone morphogenetic protein.
[Item B3] The method according to Item B1, wherein the inducer medium B for forming ventral hindgut organoid contains the fibroblast growth factor, the GSK3β inhibitor, and the bone morphogenetic protein.
[Item B4] A ventral hindgut organoid produced by the method according to any one of Items B1-B3.
[Item B5] A ventral hindgut organoid,
   expressing a ventral hindgut marker P63,
   expressing HOXA13 and CK8/KRT8, and
   not substantially expressing a dorsal hindgut marker SOX2.
[Item B5-1] A ventral hindgut organoid,
   expressing a ventral hindgut marker P63,
   expressing HOXA13 and CK8/KRT8,
   not substantially expressing a dorsal hindgut marker SOX2 and/or CDX2, or even if SOX2 and/or CDX2 are expressed, the expression level of SOX2 and/or CDX2 is less than that of SOX2 and/or CDX2 in a hindgut organoid.
[Item B6] A ventral hindgut organoid having a lumen,
   expressing at least one ventral hindgut marker selected from the group consisting of P63, ΔN63, GATA3, ISL1, and SATB2,
   expressing at least one marker selected from the group consisting of Phospho-Smad1/5/8, HOXA13, FOXA2, CK8/KRT8, and ECAD, and
   not substantially expressing at least one dorsal hindgut marker selected from the group consisting of SOX2, T, and CDX2.
[Item B7] A method for producing a bladder organoid, comprising
   culturing a ventral hindgut organoid produced by the method according to any one of Items B1-B3 or a ventral hindgut organoid according to any one of Items B4-B6 in the presence of extracellular matrix with an inducer culture C containing retinoic acid, fibroblast growth factor, and bone morphogenetic protein;
   introducing the ventral hindgut organoid into a kidney or bladder or its peripheral area of a human or a non-human mammal; or
   culturing the ventral hindgut organoid in the presence of mesenchymal stem cells or mesenchymal cells.
[Item B8] The bladder organoid produced by the method according to Item B7.
[Item B9] A bladder organoid comprising
   a first cell layer comprising cells that express UPK1B and/or UPK2 and do not substantially express P63; and
   a second cell layer localized in an outward direction relative to the first cell layer, the second cell layer comprising cells that co-express P63 and UPK1B and/or UPK2;
   wherein the bladder organoid may further comprise a third cell layer localized in an outward direction relative to the second cell layer, the third cell layer comprising cells that co-express P63 and KRT5.
[Item B10] The bladder organoid according to Item B9, comprising a lumen surrounded by the first cell layer.
[Item B11] The bladder organoid according to Item B9 or B10, comprising a fourth cell layer localized to an outward direction relative to the third cell layer, the fourth cell layer comprising stromal-like cells, and optionally further comprising a fifth cell layer localized to an outward direction relative to the fourth cell layer, the fifth cell layer comprising smooth muscle cells.
[Item B12] A method for producing a non-human mammal comprising a ventral hindgut organoid or a bladder organoid in a kidney or bladder thererof or its peripheral area, the method comprising introducing a ventral hindgut organoid produced by the method according to any one of Items B1-B3, a ventral hindgut organoid according to any one of Items B4-B6, a bladder organoid produced by the method according to Item B7, or a bladder organoid according to any one of Items B8-B11 into a kidney or bladder or its peripheral area of a non-human mammal.
[Item B 13] A non-human mammal comprising a ventral hindgut organoid produced by the method according to any one of Items B1-B3, a ventral hindgut organoid according to any one of Items B4-B6, a bladder organoid produced by the method according to Item B7, or a bladder organoid according to any one of Items B8-B 11 in a kidney or bladder thereof or its peripheral area.
[Item B14] A regenerative medicine composition for treating bladder injury or disease, comprising a ventral hindgut organoid produced by the method according to any one of Items B1-B3, a ventral hindgut organoid according to any one of Items B4-B6, a bladder organoid produced by the method according to Item B7, or a bladder organoid according to any one of Items B8-B11.
[Item B15] A method for assessing drug responsiveness to a test substance, comprising
   contacting the test substance with a ventral hindgut organoid produced by the method according to any one of Items B1-B3, a ventral hindgut organoid according to any one of Items B4-B6, a bladder organoid produced by the method according to Item B7, a bladder organoid according to any one of claims 8-11, a non-human mammal produced by the method according to Item B12, or a non-human mammal according to Item B13, and
   measuring drug responsiveness to the test substance in the ventral hindgut organoid, the bladder organoid, or the non-human mammal.
[Item B16] A method for treating a bladder injury or disease, comprising introducing a ventral hindgut organoid produced by the method according to any one of Items B1-B3, a ventral hindgut organoid according to any one of Items B4-B6, a bladder organoid produced by the method according to Item B7, or a bladder organoid according to Items B8-B11 into a kidney or bladder or its peripheral area of a mammal in need thereof.

### BRIEF DESCRIPTION OF DRAWINGS

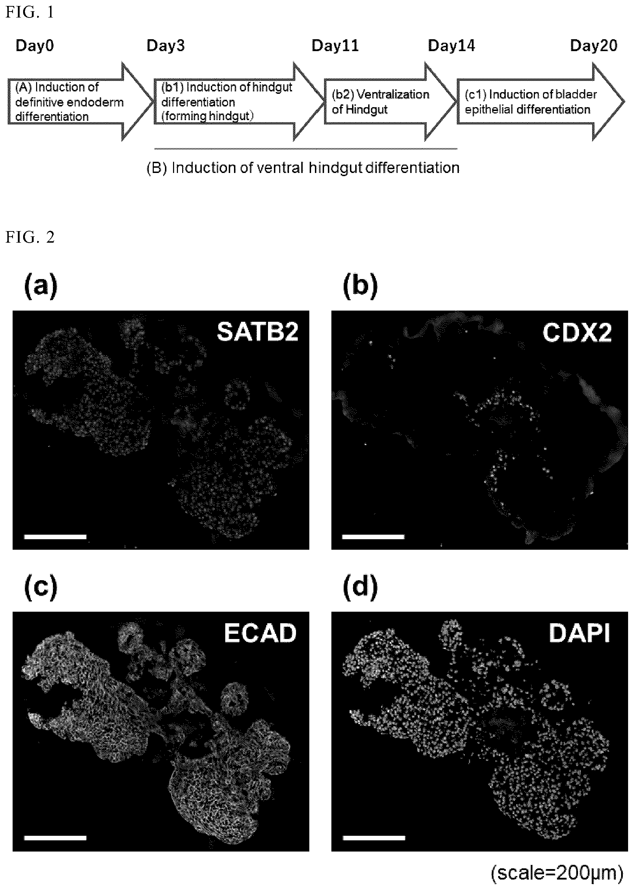

Figure 1 is a flowchart diagram illustrating an overview of the production of bladder organoids according to one embodiment.
Figure 2a is an image showing the distribution of SATB2 in gut-like organoids.
Figure 2b is an image showing the distribution of CDX2 in the gut-like organoids. Figure 2c is an image showing the distribution of ECAD in the gut-like organoids. Figure 2d is an image of DAPI staining in the gut-like organoids.

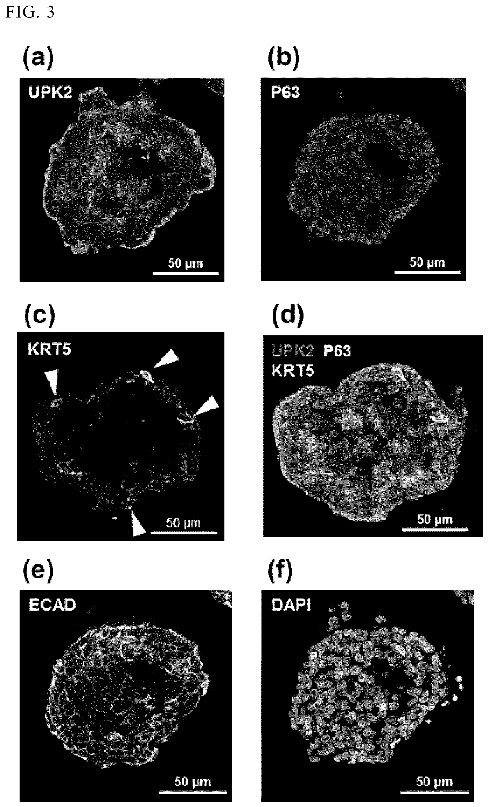

Figure 3a is an image showing the distribution of UPK2 in a bladder organoid. Note that white signals observed at the outermost periphery of the bladder organoid indicate non-specific signals derived from Matrigel^{®}. Figure 3b is an image showing the distribution of P63 in the bladder organoid. Figure 3c is an image showing the distribution of KRT5 in the bladder organoid. Figure 3d is an overlay image showing the distribution of the UPK2, P63, and KRT5 in the bladder organoid. Note that the white signals observed at the outermost periphery of the bladder organoid are non-specific signals derived from Matrigel. Figure 3e is an image showing the distribution of ECAD in the bladder organoid. Figure 3f is an image of DAPI staining in the bladder organoid.

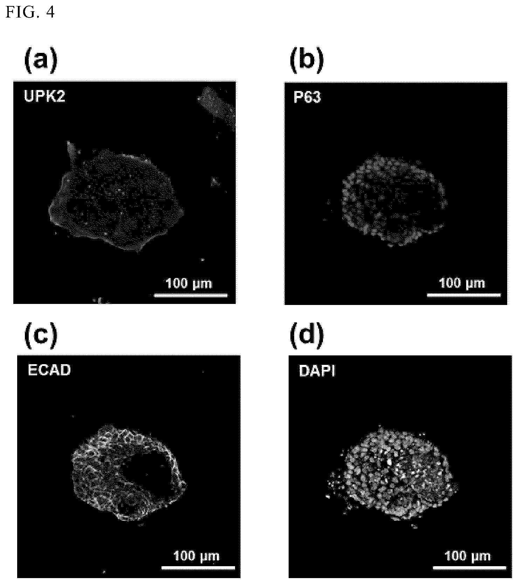

Figure 4a is an image showing the distribution of UPK2 in a cellular structure induced with an inducer medium B and a medium that is an inducer medium C minus BMP4. Note that white signals observed at the outermost periphery of the cellular structure are non-specific signals derived from Matrigel. Figure 4b is an image showing the distribution of P63 in the cellular structure induced with inducer the medium B and the medium that is the inducer medium C minus BMP4. Figure 4c is an image showing the distribution of ECAD in the cellular structure induced with the inducer medium B and the medium that is the inducer medium C minus BMP4. Figure 4d is an image of DAPI staining in the cellular structure induced with the medium that is the inducer medium C minus BMP4.

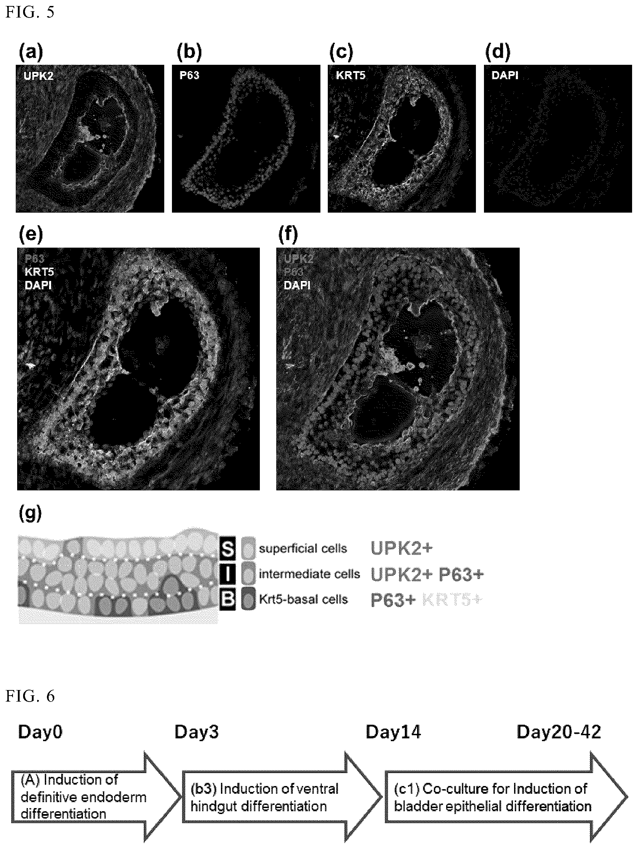

Figure 5a is an image showing the distribution of UPK2 in a bladder organoid introduced into a non-human mammal. Figure 5b is an image showing the distribution of P63 in the bladder organoid introduced into the non-human mammal. Figure 5c is an image showing the distribution of KRT5 in the bladder organoid introduced into the non-human mammal. Figure 5d is an image of DAPI staining in the bladder organoid. Figure 5e is an overlay image showing the distribution of the P63, KRT5, and DAPI staining in the bladder organoid introduced into the non-human mammal. Figure 5f is an image showing the distribution of UPK2, P63, and DAPI staining in the bladder organoid introduced into the non-human mammal. Figure 5g is a schematic diagram illustrating a layer structure of cell types in the bladder in a living body.
Figure 6 is a flowchart diagram illustrating an overview of the production of a bladder organoid according to one embodiment.

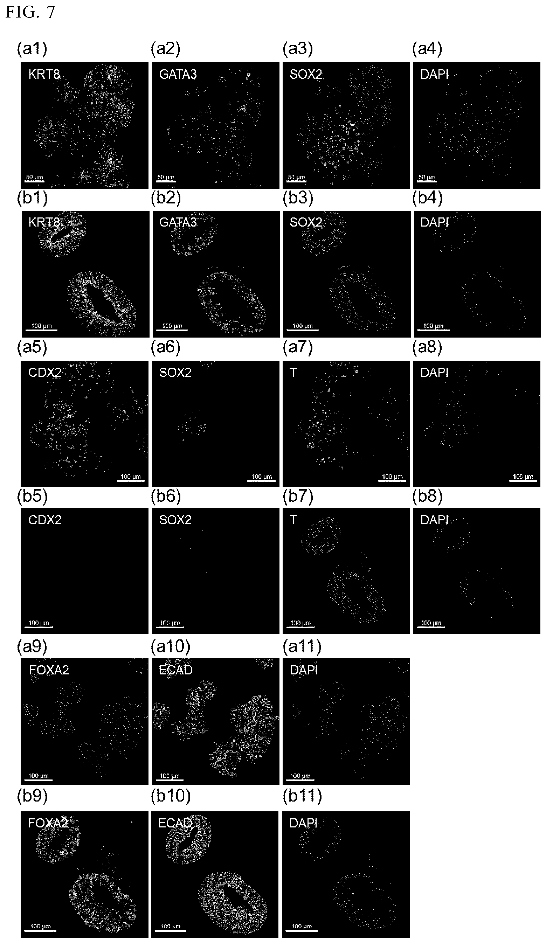

Figures 7a1-a11 are fluorescent images of hindgut organoids after inducing a hindgut differentiation in process b1 (day 11 after the induction of differentiation). Figures 7b1-b11 are fluorescent images of ventral hindgut organoids during the induction of a ventral hindgut differentiation in process b3 (day 11 after the induction of differentiation). Figures 7a1 and 7b1 are images showing the distribution of epithelial marker KRT8 in hindgut organoids and ventral hindgut organoids, respectively. Figures 7a2 and 7b2 are images showing the distribution of ventral marker GATA3 in the hindgut organoids and the ventral hindgut organoids, respectively. Figures 7a3 and 7b3 are images showing the distribution of dorsal hindgut marker SOX2 in the hindgut organoids and the ventral hindgut organoids, respectively. Figures 7a5 and 7b5 are images showing the distribution of dorsal hindgut marker CDX2 in the hindgut organoids and the ventral hindgut organoids, respectively. Figures 7a6 and 7b6 are images showing the distribution of dorsal hindgut marker SOX2 in the hindgut organoids and the ventral hindgut organoids, respectively. Figures 7a7 and 7b7 are images showing the distribution of dorsal hindgut marker T in the hindgut organoids and the ventral hindgut organoids, respectively. Figures 7a9 and 7b9 are images showing the distribution of intestinal marker FOXA2 in the hindgut organoids and the ventral hindgut organoids, respectively. Figures 7a10 and 7b10 are fluorescent images showing the distribution of epithelial marker ECAD in the hindgut organoids and the ventral hindgut organoids, respectively. Figures 7a4, 7a8, and 7a11 are images of DAPI staining in the hindgut organoids. Figures 7b4, 7b8, and 7b11 are images of DAPI staining in the ventral hindgut organoids.

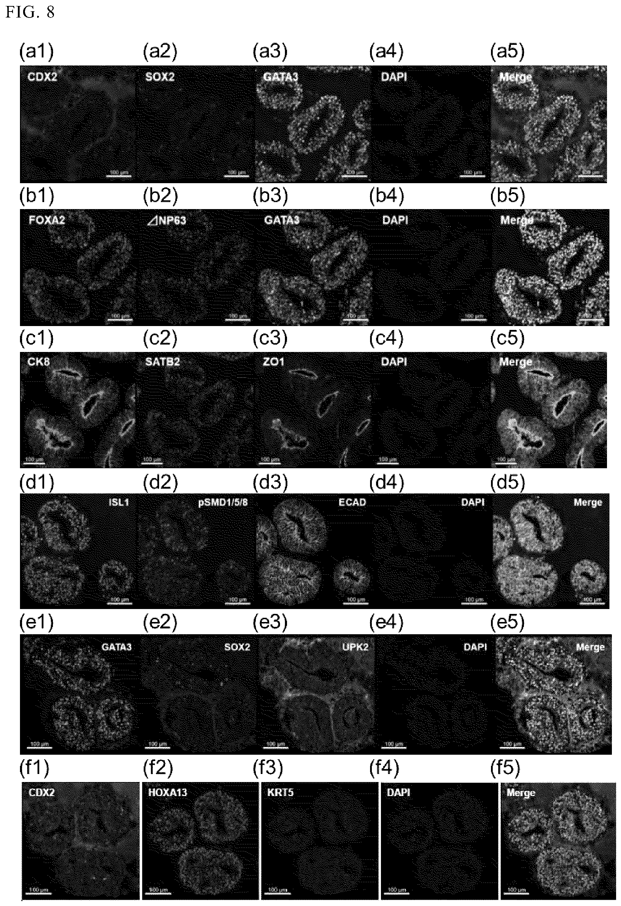

Figures 8a1 and 8a2 are fluorescent images showing the distribution of dorsal hindgut markers CDX2 and SOX2, respectively, in ventral hindgut organoids during the induction of ventral hindgut differentiation (day 14 after the induction of differentiation). Figure 8a3 is a fluorescent image showing the distribution of ventral hindgut marker GATA3. Figure 8a4 is an image of DAPI staining in the ventral hindgut organoids. Figure 8a5 is an overlay image of the CDX2, SOX2, GATA3, and DAPI staining. Figure 8b1 is a fluorescent image showing the distribution of early intestinal epithelial marker FOXA2. Figures 8b2 and 8b3 are fluorescent images showing the distribution of ventral hindgut markers ΔNP63 and GATA3, respectively, in ventral hindgut organoids. Figure 8b4 is an image of DAPI staining in the ventral hindgut organoids. Figure 8b5 is an overlay image of the FOXA2, ΔNP63, GATA3, and DAPI staining. Figure 8c1 is a fluorescent image showing the distribution of intestinal epithelial marker CK8. Figure 8c2 is a fluorescent image showing the distribution of ventral hindgut marker SATB2. Figure 8c3 is a fluorescent image showing the distribution of apical tight junction marker ZO1. Figure 8c4 is an image of DAPI staining in ventral hindgut organoids. Figure 8c5 is an overlay image of the CK8, SATB2, ZO1, and DAPI staining. Figure 8d1 is a fluorescent image showing the distribution of ventral hindgut marker ISL1. Figure 8d2 is a fluorescent image showing the distribution of ventral hindgut and Cloaca region marker, Phospho-Smad1/5/8. Figure 8d3 is a fluorescent image showing the distribution of epithelial cell marker ECAD. Figure 8d4 shows an image of DAPI staining in ventral hindgut organoids. Figure 8d5 is an overlay image of the ISL1, Phospho-Smad1/5/8, ECAD, and DAPI staining. Figure 8e1 is a fluorescent image showing the distribution of ventral hindgut marker GATA3.
Figure 8e2 is a fluorescent image showing the distribution of dorsal hindgut marker SOX2. Figure 8e3 is a fluorescent image showing the distribution of bladder epithelial cell marker UPK2. Figure 8e4 is an image of DAPI staining in ventral hindgut organoids. Figure 8e5 is an overlay image of the GATA3, SOX2, UPK2, and DAPI staining. Figure 8f1 is a fluorescent image showing the distribution of dorsal hindgut marker CDX2. Figure 8f2 is a fluorescent image showing the distribution of posterior intestinal marker HOXA13. Figure 8f3 is a fluorescent image showing the distribution of bladder epithelial cell marker KRT5. Figure 8f4 is an image of DAPI-staining in ventral hindgut organoids. Figure 8f5 is an overlay image of the CDX2, HOXA13, KRT5, and DAPI staining.

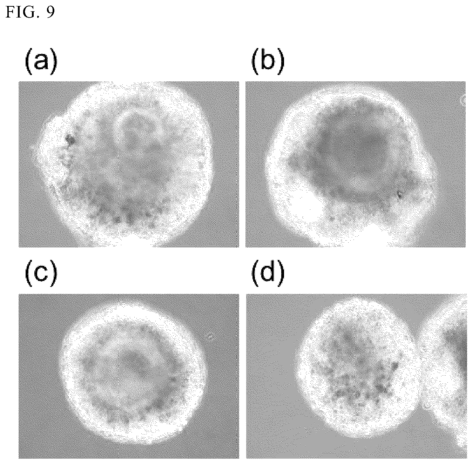

Figures 9a-d are brightfield images of bladder organoids during the induction of bladder epithelial differentiation by co-culturing ventral hindgut organoids and bladder mesenchymal cells (day 20 after the induction of differentiation).

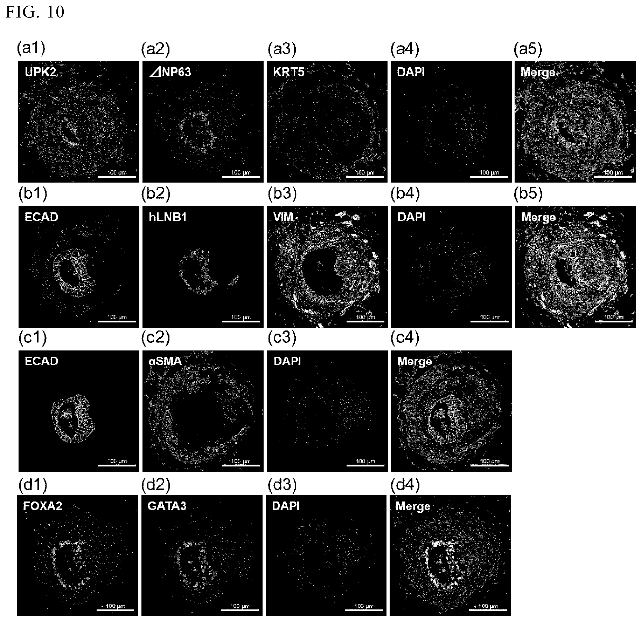

Figures 10a1-a3 are fluorescent images showing the distribution of bladder epithelial cell markers, UPK2, ΔNP63, and KRT5, respectively, in a bladder organoid during the induction of bladder organoid differentiation (day 24 after the induction of differentiation). Figure 10a4 is an image of DAPI staining in the bladder organoid. Figure 10a5 is an overlay image of the UPK2, ΔNP63, KRT5, and DAPI staining. Figure 10b1 is a fluorescent image showing the distribution of epithelial cell marker ECAD in a bladder organoid. Figure 10b2 is a fluorescent image showing the distribution of hLNB1, suggesting human-originating cells in the bladder organoid. Figure 10b3 is a fluorescent image showing the distribution of mesenchymal cell marker VIM in the bladder organoid. Figure 10b4 is an image o DAPI staining in the bladder organoid.
Figure 10b5 is an overlay image of the ECAD, hLNB1, VIM, and DAPI staining. Figure 10c1 is a fluorescent image showing the distribution of epithelial cell marker ECAD in a bladder organoid. Figure 10c2 is a fluorescent image showing the distribution of smooth muscle cells marker αSMA in the bladder organoid. Figure 10c3 is an image of DAPI staining in the bladder organoid. Figure 10c4 is an overlay image of the ECAD, αSMA, and DAPI staining. Figure 10d1 is a fluorescent image showing the distribution of developing bladder epithelial cell marker FOXA2 in a bladder organoid. Figure 10d2 is a fluorescent image showing the distribution of bladder epithelial cell marker GATA3 in the bladder organoid. Figure 10d3 is an image of DAPI staining in the bladder organoid. Figure 10d4 is an overlay image of the FOXA2, GATA3, and DAPI staining.

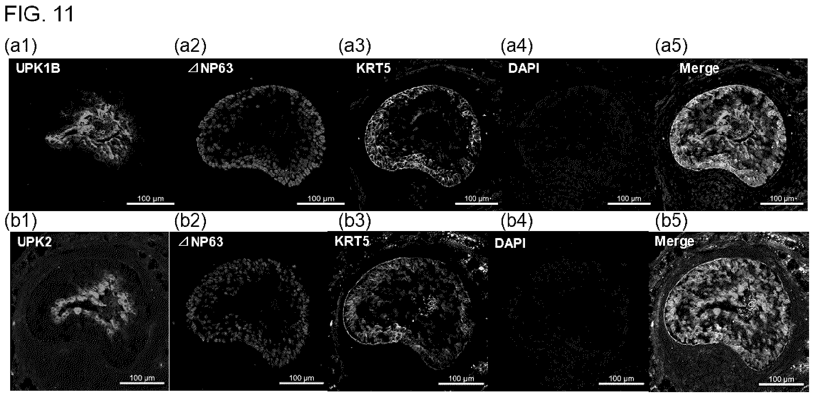

Figures 11a1 and 11b1 are fluorescent images showing the distribution of UPK1B and UPK2 in bladder organoids during the induction of bladder organoid differentiation (day 42 after the induction of differentiation), respectively. Figures 11a2 and 11b2 are fluorescent images showing the distribution of ΔNP63 in the bladder organoids. Figures 11a3 and 11b3 are fluorescent images showing the distribution of KRT5 in the bladder organoids.
Figures 11a4 and 11b4 are images of DAPI staining in the bladder organoids.
Figure 11a5 is an overlay image of the UPK1B, ΔNP63, KRT5, and DAPI staining. Figure 11b5 is an overlay image of the UPK2, ΔNP63, KRT5, and DAPI staining.

### DESCRIPTION OF EMBODIMENTS

### [Ventral hindgut organoid or method for producing the same]

One aspect of the disclosure provides a method for producing a ventral hindgut organoid. Another aspect of the disclosure provides a ventral hindgut organoid.

The term "organoid" herein refers to a three-dimensional tissue-like cell aggregate formed *in vitro* or a cell aggregate further cultured *in vivo.* For example, most cells forming the organoid can differentiate and proliferate. In this specification, the organoid expressing a specific marker refers to an organoid comprising cells expressing the specific marker at a predetermined percentage. The predetermined percentage may be, for example, not less than 3%, not less than 5%, not less than 10%, or not less than 15% of the cells forming the organoid.

"Not substantially expressing" a specific marker herein refers to the expression level of a specific marker being so low that it cannot be characterized by the marker. In an aspect, the organoid "not substantially expressing" a specific marker may be an organoid in which the expression level of the specific marker in the organoid decreased to less than 70%, less than 80%, less than 90%, or less than 95% compared to the expression level of the specific marker in an organoid to be compared. For example, the expression levels of markers can be measured by quantitative PCR or immunostaining. The expression level of a marker by quantitative PCR may be, for example, the expression level of mRNA encoding the predetermined marker. The expression level of a marker by immunostaining may be, for example, a signal intensity (e.g., fluorescence intensity) derived from a substance (e.g., fluorescent substance) capable of producing the signal, the substance being directly or indirectly bound to an antibody binding to the predetermined marker. The substance capable of generating a signal, indirectly bound to an antibody binding to a desired marker (primary antibody) may be, for example, a substance capable of generating the signal, directly bound to a secondary antibody that can bind to the primary antibody.

In an embodiment, the ventral hindgut organoid not substantially expressing SOX2 has a decreased level of SOX2 expression in the hindgut organoid to less than 70% compared to the expression level of SOX2 in a hindgut organoid. In an embodiment, the ventral hindgut organoid not substantially expressing any of at least one (e.g., one, two, or three, preferably three) selected from the group consisting of dorsal hindgut markers SOX2, T, and CDX2 has a decreased level of the dorsal hindgut marker in the hindgut organoid to less than 70% compared to the expression level of the corresponding marker in a hindgut organoid. In the embodiment, the hindgut organoid is a hindgut organoid formed by carrying out Process A and Process b1 (e.g., Process A and Process b1 in EXAMPLES) in which pluripotent stem cells, the species of which is the same as cells forming a ventral hindgut organoid, were used.

The term "ventral hindgut organoid" herein refers to an organoid that expresses at least one ventral hindgut marker according to the present disclosure and does not substantially express at least one dorsal hindgut marker according to the present disclosure or even if the dorsal hindgut marker is expressed the expression level is lower than the expression level in a hindgut organoid. The ventral hindgut organoid can be produced, for example, by the method for producing a ventral hindgut organoid according to the present disclosure. When a hindgut organoid is formed, and then the ventral hindgut organoid is ventralized to form a ventral hindgut organoid according to the method for producing a ventral hindgut organoid, the ventral hindgut organoid may be referred to herein as a "ventralized hindgut organoid." The ventral hindgut organoids may have a long diameter of not less than 80 µm, not less than 100 µm, or not less than 120 µm. For example, the ventral hindgut organoids can be used to produce bladder organoids described below. For example, the ventral hindgut organoids can be used to evaluate drug responsiveness to a test substance. For example, the ventral hindgut organoids can be used as an active ingredient of a regenerative medicine composition according to the present disclosure.

The ventral hindgut organoid according to an embodiment expresses a ventral hindgut marker P63; expresses HOXA13 and CK8/KRT8; and does not substantially express a dorsal hindgut marker SOX2. The ventral hindgut organoid according to an embodiment expresses the ventral hindgut marker P63; expresses HOXA13 and CK8/KRT8; does not substantially express the dorsal hindgut marker SOX2; and does not substantially express a dorsal hindgut marker CDX2 or even if CDX2 is expressed the expression level is less than a CDX2 expression level in a hindgut organoid. The ventral hindgut organoid according to an embodiment expresses the ventral hindgut marker P63; expresses HOXA13 and CK8/KRT8; and does not substantially express the dorsal hindgut marker SOX2 and/or CDX2 or even if SOX2 and/or CDX2 is expressed the expression level is less than a SOX2 and/or CDX2 expression level in a hindgut organoid. The ventral hindgut organoid according to an embodiment is further characterized by a higher expression level of P63 than that in a hindgut organoid. The ventral hindgut organoid according to an embodiment is further characterized in that a ratio of the expression level of CDX2 to the expression level of P63 is smaller than the ratio of the expression level of CDX2 to the expression level of P63 in a hindgut organoid. The ventral hindgut organoid according to an embodiment is further characterized in that HOXA13 is transcribed.

A ventral hindgut organoid according to an embodiment has a lumen; expresses at least one ventral hindgut marker (e.g., one kind, two kinds, three kinds, or four kinds or more) selected from the group consisting of P63, ΔN63, GATA3, ISL1, and SATB2; expresses at least one marker (e.g., one kind, two kinds, three kinds, four kinds, or five kinds or more) selected from the group consisting of Phospho-Smad1/5/8, HOXA13, FOXA2, CK8/KRT8, and ECAD; does not substantially express at least one dorsal hindgut marker (e.g., one kind, two kinds, or three kinds or more) selected from the group consisting of SOX2, T, and CDX2. The ventral hindgut organoid according to an embodiment additionally comprises a layer of cells co-expressing CK8/KRT8 and ZO1, the layer facing the above-described lumen.

The ventral hindgut organoid according to an embodiment has a lumen; expresses the ventral hindgut marker GATA3; expresses at least one marker (e.g., one kind, two kinds, or three kinds) selected from the group consisting of FOXA2, CK8/KRT8, and ECAD; and does not substantially express at least one (e.g., one kind, two kinds, or three kinds, preferably three kinds) selected from the group consisting of SOX2, T, and CDX2. The ventral hindgut organoid expresses, for example, FOXA2, CK8/KRT8, and ECAD.

The ventral hindgut organoid according to an embodiment has a lumen; comprises a layer of cells co-expressing CK8/KRT8 and ZO1, the layer facing the lumen; expresses at least one ventral hindgut marker (e.g., one kind, two kinds, or three kinds or more) selected from the group consisting of ΔN63, GATA3, ISL1, and SATB2; expresses at least one marker (e.g., one kind, two kinds, three kinds, or four kinds or more) selected from the group consisting of Phospho-Smad1/5/8, HOXA13, CK8/KRT8, FOXA2, and ECAD; and does not substantially express at least one kinds (e.g., one, two, or three, preferably three) selected from the group consisting of SOX2, T, and CDX2. The ventral hindgut organoid preferably expresses GATA3. The ventral hindgut organoid further expresses, for example, ΔN63, ISL1, and SATB2. The ventral hindgut organoid further expresses, for example, Phospho-Smad1/5/8, HOXA13, CK8/KRT8, FOXA2, and ECAD.

The term "lumen" herein refers to an inner space of a tubular or pouch-like cell structure. The lumen may, for example, be filled with a liquid. In an embodiment, the lumen of a bladder organoid is an inner space of the pouch-like cell structure.

The term "P63" regarding the ventral hindgut organoid refers to a homolog of the tumor suppressor gene P53 and a protein involved in the differentiation, proliferation, and maintenance of epithelium. P63 can be used as a bladder epithelial cell marker or a ventral hindgut/Cloaca marker. P63 expressed in cells or organoids can be detected or measured, for example, by immunostaining with anti-P63 antibody (e.g., Anti-P63 antibody rabbit monoclonal (EPR5701)).

The term "ΔNP63" herein refers to an isoform of p63 that lacks the N-terminal trans-activation domain (TN). ΔNP63 can be used as a bladder epithelial cell marker or a ventral hindgut marker. ΔNP63 expressed in cells or organoids can be detected or measured, for example, by immunostaining with anti-ΔNP63 antibody (e.g., Rabbit Anti-ΔNP63 (Cell signaling, #67825)).

The term "GATA3" herein refers to a transcription factor that binds to a target DNA sequence consisting of "GATA" of DNA and controls the switch on/off of the gene. GATA3 can be used as a ventral hindgut marker or a bladder epithelial cell marker. GATA3 expressed in cells or organoids can be detected or measured, for example, by immunostaining with anti-GATA3 antibody (e.g., Goat Anti-GATA3 (R&D Systems, #AF2605)).

The term "UPK1B" herein refers to a membrane glycoprotein involved in formation of transitional epithelial coated cells that form a uroepithelium in the urinary tract system together with uroplakin Ia, II, and III, and enhances the permeability and barrier function of the coated cells. UPK1B can be used as a bladder epithelial marker or an epithelial tissue marker. UPK1B expressed in cells or organoids can be detected or measured, for example, by immunostaining with anti-UPK1B antibody (mouse Anti-UPK1B monoclonal antibody (clone 1E1) (Sigma-Aldrich, #WH0007348M2).

The term "ISL1" herein refers to a transcription factor with LIM homeodomain that acts on the regulatory region of insulin gene expression. ISL1 can be used as a ventral hindgut marker. ISL1 expressed in cells or organoids can be detected or measured, for example, by immunostaining with anti-ISL1 antibody (Mouse Anti-ISL1&2 (DSHB, #39.4D5)).

The term "SATB2" herein refers to a DNA-binding protein that binds to an AT-rich sequence. SATB2 can be used as a colon or rectal marker or a ventral hindgut marker. SATB2 expressed in cells or organoids can be detected or measured, for example, by immunostaining with anti-SATB2 antibody (Rabbit Anti-SATB2 (CELL MARQUE, #384R-14)).

The term "CDX2" herein refers to a homeobox protein encoded by the CDX2 gene. CDX2 can be used as a midgut/hindgut marker. CDX2 expressed in cells or organoids can be detected or measured, for example, by immunostaining with anti-CDX2 antibody (e.g., Anti-CDX2 antibody mouse monoclonal (CX2-88)).

The term "SOX2" herein refers to a transcription factor essential for the maintenance of self-renewal of undifferentiated ES cells and is also called SRY (sex determining region Y)-box 2). SOX2 can be used as a dorsal hindgut marker or a lung and gastric lineage marker. SOX2 expressed in cells or organoids can be detected or measured, for example, by immunostaining with anti-SOX2 antibody (e.g., Anti-SOX2 antibody goat polyclonal (R&D Systems, #AF2018)).

The term "T" herein refers to a transcription factor that binds to a DNA sequence called palindromic T site via an N-terminal region called T-box and affects transcription of genes required for mesoderm formation and differentiation, and is also called T-box transcription factor T (TBXT). T expressed in cells or organoids can be detected or measured, for example, by immunostaining with anti-T antibody (e.g., Goat Anti-T/Brachyury antibody (R&D Systems, #AF2085)).

The term "ZO1" herein refers to a membrane phosphoprotein expressed at the tight junction of epithelial and endothelial cells. ZO1 can be used as a tight junction marker. ZO1 expressed in cells or organoids can be detected or measured, for example, by immunostaining with anti-ZO1 antibody (Goat Anti-ZO1 (ThermoFisher, #PA5-19090)).

The term "cytokeratin 8 (KRT8 or CK8)" herein refers to a subtype of keratin protein with a molecular weight of approximately 45 kD expressed in myoepithelial cells and membrane basal cells. CK8/KRT8 can be used as an intestinal epithelial marker. CK8/KRT8 expressed in cells or organoids can be detected or measured, for example, by immunostaining with an anti-Cytokeratin8 antibody rat monoclonal (TROMA-1).

The term "FOXA2" herein refers to a transcription factor that plays an important role in developmental stages and is an abbreviation for Forkhead box protein A2. FOXA2 can be used as an early intestinal epithelial marker or a developing bladder epithelial cell marker. FOXA2 can be detected or measured, for example, by immunostaining with anti-FOXA2 antibody (Mouse Anti-FOXA2 (Santa Cruz Biotechnology, #sc-101060)).

The term "ECAD" herein refers to a transmembrane glycoprotein that exists on the cell surface and is involved in cell adhesion, and is also called epithelial cadherin. ECAD can be used as an epithelial tissue marker. ECAD expressed in cells or organoids can be detected or measured, for example, by immunostaining with anti-ECAD antibody (e.g., Goat Anti-ECAD (R&D SYSTEMS, #AF648)).

The term "HOXA13" herein refers to a homeobox protein encoded by the HOXA13 gene in human. HOXA13 expressed in cells or organoids can be detected or measured, for example, by quantitative RCR.

The term "Phospho-Smad1/5/8" herein refers to transcription factors that play an important role in the intracellular TGF-β signaling pathway. Phospho-Smad1/5/8 can be used as a ventral hindgut and Cloaca region marker. Phospho-Smad1/5/8 expressed in cells or organoids can be detected or measured, for example, by immunostaining with anti-smad1/5/8 antibody (e.g., Rabbit Anti-pSmad1/5/8 (Cell signaling, #13820)).

The ventral hindgut organoid according to an embodiment of the present disclosure can be produced by a method, the method comprising Process A, culturing a pluripotent stem cell with an inducer medium A containing activin A and GSK3β inhibitor to induce differentiation into definitive endoderm cells, and Process B, culturing the definitive endoderm cells with an inducer medium B containing fibroblast growth factor, GSK3β inhibitor, and optionally further containing bone morphogenetic protein, followed by culturing them in the presence of extracellular matrix with an inducer medium B containing fibroblast growth factor, GSK3β inhibitor, and optionally further containing bone morphogenetic protein, to form the ventral hindgut organoid.

Process B in an embodiment comprises Process b1, culturing the definitive endoderm cells with an inducer medium B containing fibroblast growth factor and GSK3β inhibitor (inducer medium b), followed by culturing them in the presence of extracellular matrix with an inducer medium B (inducer medium b1) to form a hindgut organoid, and Process b2, culturing the hindgut organoid in the presence of extracellular matrix with an inducer medium B containing fibroblast growth factor, GSK3β inhibitor, and bone morphogenetic protein (inducer medium b2) to form the ventral hindgut organoid. Process B in an embodiment comprises Process b3, culturing the definitive endoderm cells in the presence of extracellular matrix with an inducer medium B containing fibroblast growth factor and GSK3β inhibitor and optionally further containing bone morphogenetic protein (inducer medium b) followed by culturing them in the presence of extracellular matrix with an inducer medium B containing fibroblast growth factor, GSK3β inhibitor, and bone morphogenetic protein (inducer medium b3) to form the ventral hindgut organoid.

The ventral hindgut organoid according to an embodiment of the present disclosure can be produced by a method, the method comprising Process A: culturing pluripotent stem cells with the inducer medium A to induce differentiation into definitive endoderm cells, Process b1, culturing the definitive endoderm cells with an inducer medium B containing fibroblast growth factor and GSK3β inhibitor (inducer medium b), followed by culturing them in the presence of extracellular matrix with an inducer medium B (inducer medium b1) to form a hindgut organoid, and Process b2, culturing the hindgut organoid in the presence of extracellular matrix with an inducer medium B containing fibroblast growth factor, GSK3β inhibitor, and bone morphogenetic protein (inducer medium b2) for form the ventral hindgut organoid.

The ventral hindgut organoid according to an embodiment of the present disclosure can be produced by a method comprising Process A: culturing pluripotent stem cells with an inducer medium A containing activin A and GSK3β inhibitor to induce differentiation into definitive endoderm cells, and Process b3: culturing the definitive endoderm cells with an inducer medium B containing fibroblast growth factor and GSK3β inhibitor, and optionally further containing bone morphogenetic protein (inducer medium b), followed by culturing them in the presence of extracellular matrix with an inducer medium B containing fibroblast growth factor, GSK3β inhibitor, and bone morphogenetic protein (inducer medium b3) to form the ventral hindgut organoid.

### Process A: Induction of differentiation into definitive endoderm cells

Process A comprises culturing a pluripotent stem cell with an inducer medium A containing activin A and GSK3β inhibitor to induce differentiation into definitive endoderm cells.

The term "pluripotent stem cell" herein refers to a stem cell capable of being differentiated into tissues originating from three germ layers (ectoderm, mesoderm, and endoderm), i.e., the stem cell having a pluripotency and can be cultured *in vitro.* Pluripotent stem cells can be established from, for example, fertilized eggs, cloned embryos, germline stem cells, or stem cells in tissues. In an embodiment, pluripotent stem cells are embryonic stem cells (ES cells), engineered pluripotent stem cells induced from somatic cells (iPS cells), embryonic carcinoma cells (EC cells), or embryonic germ cells (EG cells). Pluripotent stem cells are preferably ES cells or iPS cells.

The term "ES cell" herein refers to a pluripotent stem cell, originating from an early embryo, capable of self-replicating and being pluripotent. In an embodiment, ES cells are human ES cells.

The term "iPS cell" herein refers to a pluripotent stem cell induced from a somatic cell, engineered by initializing a somatic cell to have pluripotency resembling embryonic stem cells. iPS cells can be established, for example, by initializing differentiated cells such as fibroblasts with rendering genes such as Oct3/4, Sox2, Klf4, and Myc expressed. In an embodiment, iPS cells are human iPS cells established by initializing differentiated human cells such as fibroblasts.

An "inducer medium A" comprises a basal medium and additional agents comprising activin A and GSK3β inhibitor. For example, the inducer medium A can be prepared by adding the addition agents (solid or liquid) to the basal medium (liquid). Concentrations of the addition agents added to the inducer medium A are appropriately adjusted by a person skilled in the art, considering the animal species which provide cells used for the culturing. A "basal medium" may be a cell culture medium prepared according to known protocols or a commercially available cell culture medium. The basal medium may be, for example, Dulbecco's Modified Eagle's Medium (DMEM), Minimum Essential Medium (MEM), Basal Medium Eagle (BME), any known medium for stem cells, or any known medium for differentiating stem cells. The basal medium is preferably a medium for differentiating stem cells, e.g., STEMdiff APEL2 medium (STEMCELL Tecnologies). The culture medium for differentiating stem cells can be prepared, for example, according to Nature Protocols, vol.3, No.5, pp. 768-776, 2008. The inducer medium A may further contain protein-free medium (e.g., PFHM-II), antibiotics (e.g., penicillin/streptomycin, gentamicin), or antibiotic-antifungal mixture (e.g., Antibiotic-Antimycotic), or a combination thereof.

The term "activin A" herein refers to a factor belonging to the TGFβ superfamily and is a protein that promotes secretion of follicle-stimulating hormone (FSH) from the anterior pituitary gland. Activin A involves various functions in regulating cell differentiation, proliferation, apoptosis, and carcinogenesis. The inducer medium A may contain activin A, for example, at 10-500 ng/mL, 30-250 ng/mL, or 50-150 ng/mL.

The term "GSK3β inhibitor" herein refers to a compound that inhibits the action of serine-threonine protein kinase 3β involved in various signaling pathways including the wnt/β-catenin pathway. GSK3β inhibitor may be, for example, CHIR-99021, SB216763, CHIR-98014, Staurosporine, K252A, WNT (preferably WNT3A) or TWS119, or a combination thereof. GSK3β inhibitor is preferably CHIR-99021 or WNT (preferably WNT3A). The inducer medium A may contain GSK3β inhibitor at a concentration exerting a GSK3β inhibitory effect to the same degree as the effect exerted by CHIR-99021 at 0.1-5 µM, 0.3-2.5 µM, or 0.5-1.5 µM. A "GSK3β inhibitory activity" can be measured, for example, on the basis of the transcriptional activity by nuclear transfer of β-catenin in the presence of a predetermined amount of GSK3β inhibitor. The transcriptional activity by nuclear transfer of β-catenin can be measured according to known methods (e.g., luciferase activity assay). The transcriptional activity by nuclear transfer of β-catenin can be measured, for example, with commercially available kits (e.g., LEADING LIGHT^{®} Wnt Reporter Assay Starter kit). The term "effect to the same degree" herein refers to an effect within ± 30%, ± 20%, or ± 10% compared to the effect to be a control. In an embodiment, the effect to the same degree is an effect within ±30% compared to the effect to be a control.

The inducer medium A may contain GSK3β inhibitor (preferably CHIR-99021) at 0.1-5 µM, 0.3-2.5 µM, or 0.5-1.5 µM. The inducer medium A may contain GSK3β inhibitor at a concentration exerting a GSK3β inhibitory effect to the same degree as the effect exerted by CHIR-99021 at 0.1-5 µM, 0.3-2.5 µM, or 0.5-1.5 µM. The inducer medium A may contain WNT (preferably WNT3A) as GSK3β inhibitor at 0.2-200 ng/ml, 10-100 ng/ml, or 2-20 ng/ml. When the inducer medium A contains WNT (preferably WNT3A) as GSK3β inhibitor, the inducer medium A may contain the WNT (preferably WNT3A) at a concentration exerting a GSK3β inhibitory effect to the same degree as the effect exerted by CHIR-99021 at 0.1-5 µM, 0.3-2.5 µM, or 0.5-1.5 µM.

An inducer medium b2 described below may contain GSK3β inhibitor at a concentration exerting a GSK3β inhibitory effect to the same degree as the effect exerted by CHIR-99021 at 1-50 µM, 3-25 µM, or 5-15 µM. In an embodiment, the inducer medium b2 may contain 2-2000 ng/ml, 100-1000 ng/ml, or 20-200 ng/ml of WNT (preferably WNT3A). An inducer medium b3 described below may contain GSK3β inhibitor at a concentration exerting a GSK3β inhibitory effect to the same degree as the effect exerted by CHIR-99021 at 0.5-25 µM, 1-15 µM, or 2-8 µM. In an embodiment, the inducer medium b3 may contain 1-1000 ng/ml, 50-500 ng/ml, or 10-100 ng/ml of WNT (preferably WNT3A).

The culturing of pluripotent stem cells with the inducer medium A can be conducted under known cell culture conditions. The known cell culture conditions may be to keep at 37°C under 5% CO₂. The temperature for culturing is not limited to 37°C but may appropriately use any temperature known in the field of cell culture. The CO₂ concentration is not limited to 5% but may appropriately use any known concentration of CO₂ in the field of cell culture. The culture of pluripotent stem cells can be carried out for 2-5 days, 2-4 days, or three days.

"Definitive endoderm cells" may be induced by culturing a pluripotent stem cell with the inducer medium A containing activin A and GSK3β inhibitor.

The pluripotent stem cell cultured in the inducer medium A may be pre-cultured in any known medium for stem cells. Such pluripotent stem cells can be pre-cultured, for example, with StemFit AK02N medium (REPROCELL) in a cell culture vessel coated with iMatrix-511. The pre-cultured pluripotent stem cells may be peeled from the cell culture vessel by known methods or with commercially available reagents (e.g., TrypLE Select (Thermo Fisher Scientific)). The peeled cells may be suspended in a culture medium (e.g., StemFit AK02N (10 µM Y-27632) supplemented with iMatrix-511 (nippi) at a final concentration of 0.25 µg/cm²).

In an embodiment, the culturing of pluripotent stem cells with the inducer medium A comprises seeding a suspension of pre-cultured pluripotent stem cells (e.g., human iPS or ES cells) at 30,000-90,000 cells/cm² onto a 6-well plate and culturing them at 37°C under 5% CO₂ for one day. Next, exchange the medium for an inducer medium A, STEMdiff APEL2 medium (STEMCELL Tecnologies) (supplemented with 100 ng/ml Activin A, 1-1.25 µM CHIR99021, 2% PFHM-II, and Antibiotic-Antimycotic) and incubate for 3-5 days. The culturing induces differentiation of embryonic endoderm. Exchange the medium, for example, daily after day 2. Preferably check whether the cells express the embryonic endoderm markers FOXA2 and SOX17. A too-long term for inducing embryonic endoderm may tend to increase the proportion of foregut lineage cells (ALB+, AFP+, PDX1+) brought in the subsequent process for inducing hindgut (posteriorization). Therefore, preferably adjust the term for inducing embryonic endoderm to express FOXA2 and SOX17 and to reduce most the proportion of foregut lineage cells brought in the subsequent process for inducing hindgut.
Preferably choose the density of cells to be seeded so that the cells in a colony-like population spread outward on day 2 after the induction of differentiation and reach 100% confluent in sheet form on day 3 after the induction of differentiation. In addition, Preferably choose the condition to obtain floating spheroids at a highest number during the induction of hindgut. A low or high cell density would lead to tending to be harder to obtain floating spheroids sufficiently.

### Process b1: Induction of hindgut differentiation

Process b1 comprises culturing the definitive endoderm cells with an inducer medium B containing fibroblast growth factor and GSK3β inhibitor (inducer medium b) and then culturing them in the presence of extracellular matrix with an inducer medium B containing fibroblast growth factor and GSK3β inhibitor (inducer medium b1) to form a hindgut organoid.

An "inducer medium B" comprises a basal medium and addition agents comprising fibroblast growth factor and GSK3β inhibitor, and optionally further containing bone morphogenetic protein. The inducer medium B according to an embodiment comprises the basal medium and addition agents comprising fibroblast growth factor and GSK3β inhibitor and is used to form a spheroid from the definitive endoderm cells. The inducer medium B used to form the spheroids may also be referred to herein as "inducer medium b." The inducer medium B according to an embodiment comprises the basal medium and addition agents comprising fibroblast growth factor and GSK3β inhibitor and is used to form the hindgut organoid. The inducer medium B used to form the hindgut organoid from the spheroids is also referred to herein as "inducer medium b1". The inducer medium B used to form, from the hindgut organoid, a ventral hindgut organoid described below is also referred to herein as "inducer medium b2." The inducer medium B used to form, from the definitive endoderm cells or spheroids, a ventral hindgut organoid described below is also referred to herein as "inducer medium b3."

The inducer medium B can be prepared, for example, by adding the addition agents (solid or liquid) to the basal medium (liquid). The explanation of the basal medium for inducer medium A appropriately applies to the basal medium for inducer medium B. Concentrations of the addition agents added to the inducer medium B are appropriately adjusted by a person skilled in the art, considering the animal species which provide cells used for the culturing. The inducer medium B may further contain protein-free medium (e.g., PFHM-II), antibiotics (e.g., penicillin/streptomycin, gentamicin), or antibiotic-antifungal mixture (e.g., Antibiotic-Antimycotic), or a combination thereof.

The "inducer medium b" or "inducer medium b1" comprises the basal medium and the addition agents comprising FGF and GSK3β inhibitor. The inducer medium b or b1 can be prepared, for example, by adding the addition agents (solid or liquid) to the basal medium (liquid). The explanation of the basal medium for inducer medium A appropriately applies to the basal medium for inducer medium b or b1. In an embodiment, the inducer medium b or b1 contains fibroblast growth factor and GSK3β inhibitor and does not substantially contain bone morphogenetic protein. In an embodiment, the inducer medium b or b1 not substantially containing bone morphogenetic protein is entirely free of bone morphogenetic protein. In an embodiment, the inducer medium b not substantially containing bone morphogenetic protein may contain bone morphogenetic protein at a concentration that does not inhibit the formation of a spheroid from the definitive endoderm cells and, for example, may contain bone morphogenetic protein of less than 1 ng/ml, less than 0.5 ng/ml, or less than 0.1 ng/ml. In an embodiment, the inducer medium b1 not substantially containing bone morphogenetic protein may contain bone morphogenetic protein at a concentration that does not inhibit the formation of the hindgut organoid from the spheroid of the definitive endoderm cells and, for example, may contain bone morphogenetic protein of less than 1 ng/ml, less than 0.5 ng/ml, or less than 0.1 ng/ml.

The term "fibroblast growth factor (FGF)" herein refers to a protein with a molecular weight of 16,000-20,000 that promotes the proliferation of fibroblasts or endothelial cells. For example, FGF (e.g., FGF4) is added to the inducer medium B to suppress contamination by cells originating from the anterior endodermal. For example, FGF (e.g., FGF4) is added in the inducer medium B to form the hindgut spheroid. FGF may be, for example, FGF1, FGF2, FGF3, FGF4, FGF5, FGF6, FGF7, FGF8, FGF9, FGF10, FGF11, FGF12, FGF13, FGF14, FGF15, FGF16, FGF17, FGF18, FGF19, FGF20, FGF21, FGF22, or FGF23, or a combination thereof. FGF is preferably FGF4 or FGF7, or a combination thereof.

The inducer medium b or b1 may contain FGF (preferably FGF4) at 20-1000 ng/ml, 60-500 ng/ml, or 100-300 ng/ml. The inducer medium b or b1 may contain FGF7 at 10-500 ng/ml, 30-250 ng/ml, or 50-150 ng/ml, or FGF4 at 20-1000 ng/ml, 60-500 ng/ml, or 100-300 ng/ml. The inducer medium b or b1 contains FGF at a concentration exerting a suppression of the expression of an anterior endoderm cell marker (e.g., a hepatic lineage marker ALB, a pancreatic lineage marker PDX1, or a lung and stomach lineage marker SOX2) comparable to that in cells or organoids cultured with the inducer medium b or b1 containing FGF4 at 20-1000 ng/ml, 60-500 ng/ml, or 100-300 ng/ml. In this context, the cells to be cultured with the inducer medium b may be definitive endoderm cells induced and differentiated from a pluripotent stem cell in Process A according to the present disclosure. In this context, the cells to be cultured with the inducer medium b1 may be spheroids formed from the definitive endoderm cells in Process b1 according to the present disclosure. The expression of the anterior endoderm cell marker can be measured, for example, by immunostaining. In the immunostaining method, the expression of the anterior endoderm cell marker can be measured, for example, with a commercially available antibody. The term "suppression expression to the same degree" herein refers to an expression level within ± 30%, ± 20%, or ± 10% compared to the expression level to be a control. In an embodiment, a comparable effect is an expression level within ± 30%.

The term "ALB" herein refers to albumin, a protein consisting of about 600 amino acids with a molecular weight of about 66,000. ALB can be used, for example, as a liver lineage marker. ALB expressed in cells or organoids can be detected or measured, for example, by immunostaining with anti-albumin antibody.

The term "pancreatic and duodenal homeobox factor-1 (PDX1)" herein refers to a homeodomain protein that binds to a promoter region for the insulin gene and is involved in the specific expression of the insulin gene in pancreatic beta cells. PDX1 can be used, for example, as a pancreatic lineage marker. PDX1 expressed in cells or organoids can be detected or measured, for example, by immunostaining with anti-PDX1 antibody.

The inducer medium b or b1 may contain FGF at a concentration exerting a fibroblast proliferative effect to the same degree as the effect exerted by FGF4 at 20-1000 ng/ml, 60-500 ng/ml, or 100-300 ng/ml. The "fibroblast proliferative effect" can be measured, for example, on the basis of the amount of [³H] thymidine taken up by fibroblasts from the extracellular to the intracellular part in the presence of a predetermined amount of FGF.
Fibroblasts may be, for example, NR6R-3T3 mouse fibroblasts. The amount of thymidine taken up from the extracellular to the intracellular part can be measured according to known methods (e.g., the method described in Methods in Enzymology, Volume 109, 1985, Pages 749-773).

In an embodiment, the inducer medium b or b1 contains FGF at a concentration exerting a fibroblast proliferative effect to the same degree as the effect exhibited by FGF4 at 20-1000 ng/ml, 60-500 ng/ml, or 100-300 ng/ml.

The inducer medium b or b1 may contain GSK3β inhibitor (preferably CHIR-99021) at 1-50 µM, 3-25 µM, 5-10 µM, 0.5-16 µM, 3-12 µM, or 6-10 µM. The inducer medium b or b1 may contain WNT (preferably WNT3A) at 2-2000 ng/ml, 100-1000 ng/ml, or 20-200 ng/ml. The inducer medium b or b1 may contain GSK3β inhibitor at 1-50 µM, 3-25 µM, 5-10 µM, 0.5-16 µM, 3-12 µM, or 6-10 µM, or at a concentration exerting a GSK3β inhibitor effect to the same degree as the effect exerted by CHIR-99021 at 0.1-5 µM, 0.3-2.5 µM, or 0.5-1.5 µM. When the inducer medium b or b1 contains WNT (preferably WNT3A) as GSK3β inhibitor, the inducer medium b3 contains WNT (preferably WNT3A) at a concentration exerting a GSK3β inhibitory effect to the same degree as the effect exhibited by CHIR-99021 at 1-50 µM, 3-25 µM, 5-10 µM, 0.5-16 µM, 3-12 µM, or 6-10 µM.

The components and/or doses of the inducer medium b may be the same as or different from that of the inducer medium b1. In an embodiment, the inducer medium b has the same components and/or doses as the inducer medium b1.

A "hindgut organoid" can be formed, for example, by culturing the definitive endoderm cells with the inducer medium B according to the present disclosure. In an embodiment, the hindgut organoids can be formed by culturing the definitive endoderm cells, for example, with the inducer medium b containing FGF and GSK3β inhibitor, followed by three-dimensional culturing them in an extracellular matrix gel with the inducer medium b1 containing FGF and GSK3β inhibitor. The hindgut organoids in the extracellular matrix gel can be collected according to known methods or by a commercially available reagent. The hindgut organoids in the extracellular matrix gel can be collected, for example, by gently breaking the extracellular matrix gel with a metalloproteinase.

In an embodiment, the hindgut organoids can be formed by culturing the definitive endoderm cells, for example, with the inducer medium b containing FGF and GSK3β inhibitor, followed by culturing them on an extracellular matrix gel with the inducer medium b1 containing FGF and GSK3β inhibitor. In an embodiment, the hindgut organoids can be formed by culturing the definitive endoderm cells, for example, with the inducer medium b containing FGF and GSK3β inhibitor, followed by culturing them with the inducer medium b1 containing FGF, GSK3β inhibitor, and extracellular matrix as a dispersing component.

The term "extracellular matrix (ECM)" herein includes, but is not limited to, water, polysaccharide, elastin, integrin, and glycoprotein. Glycoprotein includes, for example, collagen, entactin (Naidogen), fibronectin, and laminin. ECM can be prepared, for example, by culturing ECM-producing cells (e.g., epithelial cells, endothelial cells, parietal endoderm-like cells, or fibroblasts) *in vitro* and then removing the ECM cells. The ECM-producing cells may be, for example, cartilage cells, which produce mainly collagen and proteoglycans; fibroblasts, which produce mainly type IV collagen, laminin, interstitial procollagen, and fibronectin; and colonic myofibroblasts, which produce mainly collagen (type I, III, and V), chondroitin sulfate proteoglycans, hyaluronic acid, fibronectin, and tenascin-C. ECM is commercially available. Commercially available extracellular matrix may be, for example, extracellular matrix protein (Invitrogen), basement membrane preparations from Engelbreth-Holm-Swarm (EHS) mouse sarcoma cells (e.g., Cultrex^{®} Basement Membrane Extract (Trevigen, Inc.) or Matrigel^{®} (Corning Inc.)). ECM may be a synthetic extracellular matrix (e.g., ProNectin (Sigma Z378666)). The extracellular matrix may be one species or a mixture of two or more species. In an embodiment, the ECM is Matrigel.

An extracellular matrix gel for forming the hindgut organoid is preferably as elastic as the gel formed with Matrigel solution whose protein concentration is 1-8 mg/mL, 1.5-6 mg/mL, 2.5 mg/mL, or 5 mg/mL. The extracellular matrix gel for forming the hindgut organoid is preferably formed with Matrigel, whose protein concentration is 1-8 mg/mL, 1.5-6 mg/mL, 1.5-3.5 mg/mL, or 4-6 mg/mL. In the case of forming a bladder organoid by conducting Process c2 described below, the extracellular matrix gel is preferably a gel having elasticity to the same degree as that formed by Matrigel solution whose protein concentration is 1-4 mg/mL, 2-3 mg/mL, or 2.5 mg/mL. The term "elasticity to the same degree" herein refers to elasticity within ± 30%, ± 20%, or ± 10% of elasticity to be a control. In an embodiment, the elasticity to the same degree is within ± 30% of elasticity to be a control. The extracellular matrix gel for forming the bladder organoid is preferably formed with Matrigel, whose protein concentration is 1-4 mg/mL, 2-3 mg/mL, or 2.5 mg/mL.

For example, the extracellular matrix as a "dispersing component" exists in a dispersed or dissolved state in the inducer medium. When existing as a dispersing component, the extracellular matrix is present, for example, at the amount of not more than 10%v/v, not more than 7%v/v, not more than 5%v/v, or not more than 2.5%v/v per volume of inducer medium. In an embodiment, the inducer medium contains extracellular matrix at the amount of 0.1-10%v/v, 0.1-7%v/v, 0.1-5%v/v, 1-10%v/v, 1-7%v/v, 1-5%v/v, 1-2.5%v/v, 2-10%v/v, 2-7%v/v, 2-5%v/v, or 2-2.5%v/v. When the extracellular matrix contains a preparation originating from an organism (e.g., basement membrane preparation from mouse sarcoma cells), the concentration in the extracellular matrix in the inducer medium is preferably low to prevent the organoids to be produced from contamination or to reduce the contamination.

Culturing the definitive endoderm cells with the inducer medium comprises, for example, culturing the definitive endoderm cells in a condition of adhering to the surface of a culture vessel. The culture in the condition is also referred to herein as two-dimensional culture. In an embodiment, a two-dimensional culture of the definitive endoderm cells with the inducer medium b results in the formation of spheroids of the cells, some of which may float in the medium. In an embodiment, a stationary culture of the definitive endoderm cells in a Low-attachment culture vessel with the inducer medium b results in the formation of spheroids of the cells, some of which can float in the medium. Spheroids suspended in the culture medium and those attached to the culture vessel can be collected by pipetting. The culture of the definitive endoderm cells with the inducer medium b can be carried out under known cell culture conditions for 3-6 days, 3-5 days, or four days.

Culturing the definitive endoderm cells with the inducer medium b1 comprises culturing spheroids of the definitive endoderm cells in the presence of extracellular matrix. The culturing of the spheroids in the presence of extracellular matrix comprises, for example, culturing the spheroids with the inducer medium b1 containing the extracellular matrix as a dispersing component. For example, the culture can be carried out by stationary culture with low-attachment culture vessels or rotary suspension culture. Culturing the spheroids in the presence of extracellular matrix as a dispersing component can result in the formation of hindgut organoids. Culturing the spheroids in the presence of extracellular matrix comprises, for example, seeding and culturing the spheroids on an extracellular matrix gel. Culturing the spheroids on the extracellular matrix gel can result in the formation of hindgut organoids. Culturing the spheroids in the presence of extracellular matrix comprises, for example, culturing the spheroids in a condition that the spheroids exist in an extracellular matrix gel. The culture in the condition is also referred to herein as a three-dimensional culture. Three-dimensional culturing of the spheroids of the definitive endoderm cells can result in the formation of hindgut organoids. The culture of spheroids of the definitive endoderm cells with the inducer medium b1 in the presence of extracellular matrix can be carried out, for example, under known cell culture conditions for 3-6 days, 3-5 days, or four days.

In an embodiment, Process b1 comprises culturing the definitive endoderm cells with the inducer medium b to form spheroids of the definitive endoderm cells, forming an extracellular matrix gel containing the spheroids of the definitive endoderm cells, and then culturing the spheroids in the extracellular matrix gel with the inducer medium b1 to form hindgut organoids. In an embodiment, Process b1 comprises culturing the definitive endoderm cells with the inducer medium b to form spheroids of the definitive endoderm cells and then culturing the spheroids on an extracellular matrix gel with the inducer medium b1 to form hindgut organoids. In an embodiment, Process b1 comprises culturing the definitive endoderm cells with the inducer medium b to form spheroids of the definitive endoderm cells and then culturing the spheroids in the presence of extracellular matrix as a dispersing component with the inducer medium b1 to form hindgut organoids.

In an embodiment, Process b1 comprises culturing the definitive endoderm cells in an inducer medium b, STEMdiff APEL2 medium (200 ng/ml FGF4, 8 µM CHIR99021, 1 µg/mL Heparin, 2% PFHM-II, Antibiotic-Antimycotic) for 1-9 days to form spheroids of the definitive endoderm cells. During the spheroid formation, collect the suspended spheroids when the spheroids float the most and start 3-dimensional culture. Start the shaking culture at 100 rpm the day before the spheroids float the most. The next day, collect the floating spheroids into a 35mm dish. Then, collect by pipetting the remaining spheroids in the well from which the spheroids were collected. Keep the collected spheroids on ice. Dilute Matrigel Growth Factor Reduced (Corning) in an inducer medium b1 (2.5-10mg/mL), add it at 50 µL/well onto Cell Culture Insert Transparent PET Membrane 24 well 8.0 µm pore-size (corning), and incubate it at 37°C for 30min-60min to gel. Next, add and mix the collected spheroids of 30-100 to the Matrigel solution of 50 µL and add the total volume thereof on top of the gelled Matrigel. Incubate it at 37°C under 5% CO₂ for 30 min-60 min to gel, add the inducer medium b1 onto the top of the Cell Culture Insert at 200 µL and under it at 300 µL, and culture at 37°C under 5% CO₂. Preferably exchange the medium daily in 2-D culture and every two days in 3-D culture. During induction of hindgut differentiation, it is desirable to check that the cells co-express the midgut/hindgut markers, FOXA2 and CDX2. The culturing may continue with the inducer medium b1 until the expression of P63, which is expressed in the hindgut/Cloaca region, is detected.

### Process b2: Ventralization of hindgut

Process b2 comprises culturing the hindgut organoid in the presence of extracellular matrix with an inducer medium B containing fibroblast growth factor, GSK3β inhibitor, and bone morphogenetic protein (inducer medium b2) to form a ventral hindgut organoid.

An "inducer medium b2" comprises a basal medium and addition agents comprising FGF, GSK3β inhibitor, and bone morphogenetic factor. For example, the inducer medium b2 can be prepared by adding the addition agents (solid or liquid) to the basal medium (liquid). The explanation of the basal medium for inducer medium A appropriately applies to the basal medium for inducer medium b2.

The inducer medium b2 may contain FGF (preferably FGF4) at 20-1000 ng/ml, 60-500 ng/ml, or 100-300 ng/ml. The inducer medium b2 may contain FGF7 at 10-500 ng/ml, 30-250 ng/ml, or 50-150 ng/ml, or FGF4 at 20-1000 ng/ml, 60-500 ng/ml, or 100-300 ng/ml. The inducer medium b2 may contain FGF at a concentration exerting a suppression of the expression of anterior endoderm cell marker (e.g., a hepatic lineage marker ALB, a pancreatic lineage marker PDX1, or a lung and stomach lineage marker SOX2) to the same degree as the expression suppression in the organoid cultured with the inducer medium b2 containing FGF4 at 20-1000 ng/ml, 60-500 ng/ml, or 100-300 ng/ml. In this context, the organoid to be cultured with the inducer medium b2 may be a hindgut organoid induced and differentiated from the definitive endoderm cells in Process b1 according to the present disclosure. The inducer medium b2 may contain FGF at a concentration exerting a fibroblast growth effect to the same degree as the effect exerted by FGF4 at 20-1000 ng/ml, 60-500 ng/ml, or 100-300 ng/ml. In an embodiment, the inducer medium b2 may contain FGF at a concentration exerting a fibroblast growth effect to the same degree as the effect exerted by FGF4 at 20-1000 ng/ml, 60-500 ng/ml, or 100-300 ng/ml.

The inducer medium b2 may contain GSK3β inhibitor (preferably CHIR-99021) at 1-50 µM, 3-25 µM, 5-10 µM, 0.5-16 µM, 3-12 µM, or 6-10 µM. The inducer medium b2 may contain WNT (preferably WNT3A) as GSK3β inhibitor at 2-2000 ng/ml, 100-1000 ng/ml, or 20-200 ng/ml. The inducer medium b2 may contain GSK3β inhibitor at a concentration exerting a GSK3β inhibitory effect to the same degree as the effect exerted by CHIR-99021 at 1-50µM, 3-25µM, or 5-10µM. When the inducer medium b2 contains WNT (preferably WNT3A) as GSK3β inhibitor, the inducer medium b2 may contain WNT (preferably WNT3A) at a concentration exerting a GSK3β inhibitory effect to the same degree as the effect exerted by CHIR-99021 at 1-50µM, 3-25µM, or 5-10µM.

The term "bone morphogenetic protein (BMP)" herein refers to a protein belonging to the transforming growth factor beta superfamily and inducing ectopic bone formation. BMP is added in the inducer medium B (e.g., the inducer medium b2 and b3) to induce the ventral hindgut organoid. BMP may be, for example, BMP2, BMP4, BMP7, or a combination thereof. BMP is preferably BMP4. The inducer medium b2 may contain BMP (e.g., BMP4) at 3-150 ng/ml, 9-75 ng/ml, or 15-45 ng/ml.

The inducer medium b2 may contain BMP at a concentration exerting an induction effect of alkaline phosphatase production to the same degree as the effect exerted by BMP4 at 3-150 ng/ml, 9-75 ng/ml, or 15-45 ng/ml. The "induction effect of alkaline phosphatase production" can be measured, for example, on the basis of the amount of alkaline phosphatase produced in chondrogenic cells in the presence of a predetermined amount of BMP. For example, mouse cells forming cartilage can be used as chondrogenic cells. The amount of alkaline phosphatase production can be measured according to known methods (e.g., the method described in Biochemical and Biophysical Research Communications, Volume 315, Issue 2, Pages 272-280).

The inducer medium b2 may contain BMPs at a concentration expressing a ventral hindgut marker (e.g., GATA3, P63, or HOXA13) to the same degree as the expression thereof in cells or organoids cultured with the inducer medium b2 containing BMP4 at 3-150 ng/ml, 9-75 ng/ml, or 15-45 ng/ml. In this context, the cells to be cultured with the inducer medium b2 may be the hindgut organoids induced and differentiated from the definitive endoderm cells in Process b1 according to the present disclosure. The expression of the ventral hindgut marker can be measured, for example, by immunostaining. In the immunostaining method, the expression of the ventral hindgut marker can be measured with antibodies for each marker described in the present disclosure. The term "expression to the same degree" herein refers to an expression within ± 30%, ± 20%, or ± 10% compared to the expression to be a control. In an embodiment, the expression to the same degree is an expression within ±30% compared to the expression to be a control.

In an embodiment, the inducer medium b2 contains BMP at a concentration exerting an induction effect of alkaline phosphatase production to the same degree as the effect exerted by BMP4 at 3-150 ng/ml, 9-75 ng/ml, or 15-45 ng/ml.

The components and/or doses between the inducer medium b, the inducer medium b1, and the inducer medium b2 from which BPM is removed may be the same or different. In an embodiment, the components and/or doses between the inducer medium b, the inducer medium b1, and the inducer medium b2 from which BPM is removed are the same.

The explanation for the culture of the spheroid of definitive endoderm cells with the inducer medium b1 in the presence of extracellular matrix described above appropriately applies to the culture of the hindgut organoid with the inducer medium b2 in the presence of extracellular matrix. For example, the culturing of a hindgut organoid with the inducer medium b2 in the presence of extracellular matrix can be carried out under known cell culture conditions. The known cell culture conditions may be to keep at 37°C under 5% CO₂. The temperature for culturing is not limited to 37°C but may appropriately use any temperature known in the field of cell culture. The CO₂ concentration is not limited to 5% but may appropriately use any known concentration of CO₂ in the field of cell culture. The culture of the hindgut organoid can be carried out for 3-6 days, 3-5 days, or four days.

The forms of extracellular matrix (gel form or form of dispersing component) in Process b1 and Process b2 may be the same or different. In an embodiment, the forms of the extracellular matrix in Process b1 and Process b2 are the same. In an embodiment, an extracellular matrix gel including hindgut organoids may be the extracellular matrix gel formed in Process b1 or an extracellular matrix gel newly formed by collecting the hindgut organoids from the extracellular matrix gel after Process b1 and forming it in which the collected hind organoids are included. For the simplicity of operation, the extracellular matrix gel including the hindgut organoid in Process b2 is the extracellular matrix gel formed in Process b1.

In an embodiment, Process b2 comprises culturing the hindgut organoids in an extracellular matrix gel with the inducer medium b2 to form ventral hindgut organoids. In an embodiment, Process b2 comprises culturing the hindgut organoids on an extracellular matrix gel with the inducer medium b2 to form ventral hindgut organoids. In an embodiment, Process b2 comprises culturing the hindgut organoids in the presence of extracellular matrix as a dispersing component with the inducer medium b2 to form ventral hindgut organoids.

In an embodiment, the culturing of hindgut organoids with the inducer medium b2 comprises culturing the hindgut organoids in an extracellular matrix gel with an inducer medium b2, STEMdiff APEL2 medium (30 ng/ml BMP4, 200 ng/ml FGF4, 8 µM CHIR99021, 1 µg/mL Heparin, 2% PFHM-II, Antibiotic-Antimycotic) or STEMdiff APEL2 medium (30 ng/ml BMP4, 100 ng/ml FGF7, 200 ng/ml FGF4, 8 µM CHIR99021, 1 µg/mL Heparin, 2% PFHM-II, Antibiotic-Antimycotic) for 3-6 days to ventralize the hindgut. Add the inducer medium b2 onto Cell Culture Insert gel at 200 µL and under it at 300 µL and exchange the medium every two days. Preferably keep the ventralization until the expressions of the dorsal hindgut markers SOX2 and CDX2 decrease and the ventral hindgut/Cloaca marker P63 is strongly expressed in the cells. Insufficient posteriorization and ventralization lead to contamination by midgut and dorsal intestinal lineage cells. In this case, prolong the period for inducing or ventralizing the hindgut.

### Process b3: Formation of ventral hindgut organoid

Process b3 comprises culturing the definitive endoderm cells with an inducer medium B containing fibroblast growth factor, GSK3β inhibitor, and optionally further containing bone morphogenetic protein and then culturing them in the presence of extracellular matrix with an inducer medium B containing fibroblast growth factor, GSK3β inhibitor, and bone morphogenetic protein (inducer medium b3), to form a ventral hindgut organoid. In an embodiment, Process b3 comprises culturing the definitive endoderm cells with the inducer medium B to form spheroids of the definitive endoderm cells and then culturing them in the presence of extracellular matrix with the inducer medium b3 to form ventral hindgut organoids. In an embodiment, the components and/or doses of the inducer medium B for forming spheroids of the definitive endoderm cells may be the same as that of the inducer medium b3. In this context, Process b3 comprises culturing the definitive endoderm cells with an inducer medium B containing fibroblast growth factor, GSK3β inhibitor, and bone morphogenetic protein (inducer medium b3), and then culturing them in the presence of extracellular matrix with the inducer medium B (inducer medium b3) to form ventral hindgut organoids.

The ventral hindgut organoids according to an embodiment of the present disclosure can be produced by a method comprising Process A: culturing pluripotent stem cells with the inducer medium A containing activin A and GSK3β inhibitor to induce differentiation into definitive endoderm cells, and Process b3: culturing the definitive endoderm cells with an inducer medium B containing fibroblast growth factor, GSK3β inhibitor, and optionally bone morphogenetic protein (inducer medium b3) and then culturing them in the presence of extracellular matrix with the inducer medium B (inducer medium b3) to form ventral hindgut organoids.

An "inducer medium b3" comprises a basal medium and addition agents comprising FGF, GSK3β inhibitor, and bone morphogenetic factor. For example, the inducer medium b3 can be prepared by adding the addition agents (solid or liquid) to the basal medium (liquid). The explanation of the basal medium for inducer medium A appropriately applies to the basal medium for inducer medium b3.

The inducer medium b3 may contain FGF (preferably FGF4) at 20-1000 ng/ml, 60-500 ng/ml, or 100-300 ng/ml. The inducer medium b3 may contain FGF7 at 10-500 ng/ml, 30-250 ng/ml, or 50-150 ng/ml, or FGF4 at 20-1000 ng/ml, 60-500 ng/ml, or 100-300 ng/ml. The inducer medium b3 may contain GSK3β inhibitor (preferably CHIR-99021) at 0.5-25 µM, 1-15 µM, 2-8 µM, 0.1-10 µM, 0.5-9 µM, 1-8 µM, 2-6 µM, or 3-5 µM. The inducer medium b3 may contain bone morphogenetic factor (preferably BMP4) at 0.1-100 ng/ml, 0.5-80 ng/ml, 1-60 ng/ml, 2-40 ng/ml, or 5-20 ng/ml.

The inducer medium b3 may contain FGF at a concentration exerting a suppression of the expression of anterior endoderm cell marker (e.g., a hepatic lineage marker ALB, a pancreatic lineage marker PDX1, or a lung and stomach lineage marker SOX2) to the same degree as the expression suppression exerted in the cells or organoid cultured with the inducer medium b3 containing FGF4 at 20-1000 ng/ml, 60-500 ng/ml, or 100-300 ng/ml. In this context, the cells to be cultured with the inducer medium b3 may be definitive endoderm cells induced and differentiated from pluripotent stem cells in Process A according to the present disclosure. In this context, the organoid to be cultured with the inducer medium b3 may be a hindgut organoid induced and differentiated from the definitive endoderm cells in Process b1 according to the present disclosure. The inducer medium b3 may contain FGF at a concentration exerting a fibroblast growth effect to the same degree as the effect exerted by FGF4 at 20-1000 ng/ml, 60-500 ng/ml, or 100-300 ng/ml. In an embodiment, the inducer medium b3 may contain FGF at a concentration exerting a fibroblast growth effect to the same degree as the effect exerted by FGF4 at 20-1000 ng/ml, 60-500 ng/ml, or 100-300 ng/ml.

The inducer medium b3 may contain WNT (preferably WNT3A) as a GSK3β inhibitor at 1-1000 ng/ml, 50-500 ng/ml, or 10-100 ng/ml. The inducer medium b3 may contain GSK3β at a concentration exerting a GSK3β inhibitory effect to the same degree as the effect exerted by CHIR-99021 at 0.5-25 µM, 1-15 µM, or 2-8 µM. When the inducer medium b3 contains WNT (preferably WNT3A) as a GSK3β inhibitor, the inducer medium b3 may contain WNT (preferably WNT3A) at a concentration exerting a GSK3β inhibitory effect to the same degree as the effect exerted by CHIR-99021 at 0.5-25 µM, 1-15 µM, or 2-8 µM.

The inducer medium b3 may contain BMP at a concentration exerting an induction effect of alkaline phosphatase production to the same degree as the effect exerted by BMP4 at 0.1-100 ng/ml, 0.5-80 ng/ml, 1-60 ng/ml, 2-40 ng/ml, or 5-20 ng/ml. The inducer medium b3 may contain BMP at a concentration expressing a ventral hindgut marker (e.g., GATA3, P63, or HOXA13) in the cells or organoid cultured with the inducer medium b3 containing BMP4 at 0.1-100 ng/ml, 0.5-80 ng/ml, 1-60 ng/ml, 2-40 ng/ml, or 5-20 ng/ml. In an embodiment, the inducer medium b3 contains BMP at a concentration exerting an induction effect of alkaline phosphatase production to the same degree as the effect exerted by BMP4 at 0.1-100 ng/ml, 0.5-80 ng/ml, 1-60 ng/ml, 2-40 ng/ml, or 5-20 ng/ml.

In an embodiment, Process b3 comprises culturing the definitive endoderm cells with an inducer medium B (preferably inducer medium b3) to form spheroids of the definitive endoderm cells, forming an extracellular matrix gel including the spheroid of the definitive endoderm cells, and then culturing the spheroids in the extracellular matrix gel with the inducer medium b3 to form ventral hindgut organoids. In an embodiment, Process b3 comprises culturing the definitive endoderm cells with an inducer medium B (preferably inducer medium b3) to form spheroids of the definitive endoderm cells and then culturing the spheroids on an extracellular matrix gel with the inducer medium b3 to form ventral hindgut organoids. In an embodiment, Process b3 comprises culturing the definitive endoderm cells with an inducer medium B (preferably inducer medium b3) to form spheroids of the definitive endoderm cells and then culturing the spheroids in the presence of extracellular matrix as a dispersing component with the in inducer medium b3 to form ventral hindgut organoids.

In an embodiment, Process b3 comprises culturing definitive endoderm cells in an inducing medium b3, STEMdiff APEL2 medium (10- 30 ng/ml BMP4, 200 ng/ml FGF4, 4 µM CHIR99021, 1 µg/mL Heparin, 2% PFHM-II, Antibiotic-Antimycotic) for 8-11 days to form spheroids of the definitive endoderm cells. During the spheroid formation, collect the suspended spheroids when the spheroids float the most and start 3-dimensional culture. The next day, collect the floating spheroids into a 35mm dish. Then, collect the spheroids remaining in the well, from which the suspended spheroids were collected, by pipetting a medium over them to float. Keep the collected spheroids on ice. Dilute Matrigel Growth Factor Reduced (Corning) in an inducer medium b3 (25-100% concentration), add it at 50 µL/well onto Cell Culture Insert Transparent PET Membrane 24 well 8.0 µm pore-size (corning), and incubate it at 37°C for 30min-60min to gel. Next, add and mix the collected spheroids of 30-100 to the Matrigel solution of 50 µL and add the total volume thereof onto the top of the gelled Matrigel. Incubate it at 37°C under 5% CO₂ for 30 min-60 min to gel, add the inducer medium b3 onto the top of the Cell Culture Insert at 200 µL and under it at 300 µL, and culture at 37°C under 5% CO₂. Preferably exchange the medium daily in 2-D culture and every two days in 3-D culture. During induction of ventral hindgut differentiation, the cells are cultured with the inducer medium b3 until they express the ventral hindgut/Cloaca markers GATA3 and P63, and the dorsal midgut/hindgut markers CDX2, SOX2, and T are expressed at a reduced level or not expressed. The obtained ventral hindgut organoids have a round shape and a luminal structure. Insufficient posteriorization and ventralization of the organoids lead to the appearance of midgut and dorsal intestinal tract lineage cells. In this case, prolong the period for culturing with the inducer medium b3 or increase BMP4 concentration in the inducer medium b3.

The explanation of each term for each process in the induction of differentiation into definitive endoderm cells (Process A) and the formation of ventral hindgut organoid (Process B), as well as the explanation of embodiments including culture conditions appropriately apply to the corresponding term and processes in their embodiments, respectively.

### [Bladder organoid or method for producing the same]

An aspect of the present disclosure provides a method for producing a bladder organoid. Another aspect of the present disclosure provides a bladder organoid.

The term "bladder organoid" herein refers to an organoid having at least two cell layers.
The at least two cell layers comprise a first cell layer comprising cells that express UPK1B and/or UPK2 and do not substantially express P63; and a second cell layer localized in an outward direction relative to the first cell layer, the second cell layer comprising cells that co-express P63 and UPK1B and/or UPK2. The bladder organoid according to an embodiment comprises a first cell layer comprising cells that express UPK1B and/or UPK2 and do not substantially express P63; a second cell layer localized in an outward direction relative to the first cell layer, the second cell layer comprising cells that co-express P63 and UPK1B and/or UPK2; and a third cell layer localized in an outward direction relative to the second cell layer, the third cell layer comprising cells that co-express P63 and KRT5. The bladder organoid may be specified in another expression as follows: the bladder organoid comprising, for example, a first cell layer comprising cells co-expressing P63 and KRT5, localized along the outermost periphery; a second cell layer comprising cells co-expressing P63 and UPK1B and/or UPK2, localized in an inward direction relative to the first cell layer; and a third cell layer comprising cells expressing UPK1B and/or UPK2 but not substantially expressing P63, localized in an inward direction relative to the second cell layer. Each cell layer thus identified is also referred to herein as follows: the first cell layer (basal-cell layer), the second cell layer (intermediate-cell layer), and the third cell layer (superficial-cell layer).

The bladder organoid can be produced, for example, by the method for producing a bladder organoid according to the present disclosure. A bladder organoid, formed by forming a ventral hindgut organoid and culturing the ventral hindgut organoid *in vitro* in the method for producing the bladder organoid, may be referred to herein as a "bladder-like organoid." The bladder organoid may have not less than 80 µm, not less than 100 µm, not less than 120 µm, not less than 150 µm, or not less than 200 µm in the major axis. For example, the bladder organoid can be used to assess drug responsiveness to a test substance. The bladder organoid can be used, for example, as an active ingredient of a regenerative medicine composition according to the present disclosure.

The bladder organoid according to an embodiment does not have a lumen structure; comprises a first cell layer comprising cells that express UPK1B and/or UPK2 and do not substantially express P63; a second cell layer localized in an outward direction relative to the first cell layer, the second cell layer comprising cells that co-express P63 and UPK1B and/or UPK2; and a third cell layer localized in an outward direction relative to the second cell layer, the third cell layer comprising cells that co-express P63 and KRT5. In an embodiment, the bladder organoid comprises a fourth cell layer localized to an outward direction relative to the third cell layer, the fourth cell layer comprising stromal-like cells. In an embodiment, the bladder organoid further comprises a fifth cell layer localized to an outward direction relative to the fourth cell layer, the fifth cell layer comprising smooth muscle cells.

The bladder organoid according to an embodiment has a lumen; a first cell layer comprising cells that express UPK1B and/or UPK2 and do not substantially express P63, the first cell layer facing the lumen; and a second cell layer localized in an outward direction relative to the first cell layer, the second cell layer comprising cells that co-express P63 and UPK1B and/or UPK2. The first cell layer surrounds the lumen or exists facing the lumen. The bladder organoid according to an embodiment has a lumen; comprises a first cell layer comprising cells that express UPK1B and/or UPK2 and do not substantially express P63, the first cell layer facing the lumen; a second cell layer localized in an outward direction relative to the first cell layer, the second cell layer comprising cells that co-express P63 and UPK1B and/or UPK2; and a third cell layer localized in an outward direction relative to the second cell layer, the third cell layer comprising cells that co-express P63 and KRT5. The first cell layer surrounds the lumen or exits facing the lumen. In an embodiment, the bladder organoid comprises a fourth cell layer localized to an outward direction relative to the third cell layer, the fourth cell layer comprising stromal-like cells. In an embodiment, the bladder organoid further comprises a fifth cell layer localized to an outward direction relative to the fourth cell layer, the fifth cell layer comprising smooth muscle cells.

The bladder organoid is characterized by expressing at least one (e.g., one kind, two kinds, or three kinds or more) ventral hindgut marker selected from the group consisting of ΔN63, GATA3, ISL1, and SATB2; expressing at least one (e.g., one kind, two kinds, three kinds, four kinds, five kinds, or six kinds or more) marker selected from the group consisting of Smad1/5/8, HOXA13, FOXA2, CK8/KRT8, ECAD, and UPK1B; and not substantially expressing at least one kind (e.g., one kind, two kinds, or three kinds, preferably three kinds) selected from the group consisting of SOX2, T, and CDX2. The bladder organoid according to an embodiment further expresses either or both ΔNP63 and GATA3. The bladder organoid expresses at least one marker (e.g., one kind, two kinds, or three kinds or more) selected from the group consisting of Smad1/5/8, FOXA2, ECAD, and UPK1B. The bladder organoid substantially expresses neither dorsal hindgut markers SOX2 nor CDX2.

The term "uroplakin 2 (UPK2)" herein refers to a membrane glycoprotein with a molecular weight of about 15 kDa, which is involved in formation of transitional epithelial coated cells that form a uroepithelium in the urinary tract system together with uroplakin Ia, II, and III, and enhances the permeability and barrier function of the coated cells. UPK2 can be used as a bladder epithelial marker. UPK2 expressed in cells or organoids can be detected or measured, for example, by immunostaining with anti-UPK2 antibody (e.g., Mouse Anti-Uroplakin II (BIOCARE MEDICAL, #ACR3051C)).

The term "P63" regarding the bladder organoid can be used as a bladder epithelial marker. P63 expressed in cells or organoids can be detected or measured, for example, by immunostaining with anti-P63 antibody (e.g., Rabbit Anti-P63 (Abcam, #ab 124762)).

The term "keratin 5 (KRT5)" herein refers to a protein encoded by the KRT5 gene, which dimerizes with keratin 14 to form intermediate filaments forming the cytoskeleton of basal epithelial cells. KRT5 can be used as a bladder epithelial marker. KRT5 expressed in cells or organoids can be detected or measured, for example, by immunostaining with anti-KRT5 antibody (e.g., Chicken Anti-Keratin5 (Bio Legend, #905903)).

The term "cell layer" herein refers to a stack of cell populations in which a specific cell type predominantly exists. For example, the cell layer may have a stack of cell populations in which one specific cell type exists not less than 70%, 75%, 80%, 85%, or 90% of the cell population. In one example, the first, second, or third cell layer may be a region of one cell type-population in which one specific cell type predominantly exists and can be distinguished from other cell populations.

In one example, the cell layer includes not less than 70%, not less than 75%, not less than 80%, preferably not less than 85%, and more preferably not less than 90% of cells that express UPK1B and/or UPK2 and do not substantially express P63. In one example, the cell layer consists essentially of cells that express UPK1B and/or UPK2 and do not substantially express p63. In one example, a cell layer, comprising or consisting essentially of cells that express UPK1B and/or UPK2 and do not substantially express P63, has a pouch-like shape and a lumen. In the example, the lumen is surrounded by the cell layer. In the examples, the cell layer faces the lumen. In one example, the cell layer, comprising or consisting essentially of cells that express UPK1B and/or UPK2 and do not substantially express P63, faces the lumen and stacks in an inward direction relative to the cell layer comprising or consisting essentially of cells that co-express P63 and UPK1B and/or UPK2 described below.

In one example, the cell layer includes not less than 70%, not less than 75%, not less than 80%, preferably not less than 85%, more preferably not less than 90% of cells co-expressing P63 and UPK1B and/or UPK2. In one example, the cell layer consists essentially of cells co-expressing P63 and UPK1B and/or UPK2. In one example, the cell layer comprising or consisting essentially of cells co-expressing P63 and UPK1B and/or UPK2 has a pouch-like shape. In one example, the cell layer overlays in an outward direction relative to the cell layer comprising or consisting essentially of cells that express UPK1B and/or UPK2 and do not substantially express P63. In the example, the cells co-expressing P63 and UPK1B and/or UPK2 are localized in an outward direction relative to the cell layer comprising or consisting essentially of cells expressing UPK1B and/or UPK2. In one example, the cell layer comprising or consisting essentially of cells co-expressing P63 and UPK1B and/or UPK2 exists between the cell layer comprising or consisting essentially of cells that express UPK1B and/or UPK2 and do not substantially express P63 and the cell layer comprising or consisting essentially of cells co-expressing P63 and KRT5 described below.

In one example, the cell layer includes not less than 70%, not less than 75%, not less than 80%, preferably not less than 85%, and more preferably not less than 90% of cells co-expressing P63 and KRT5. In one example, the cell layer consists essentially of cells co-expressing P63 and KRT5. In one example, the cell layer comprising or consisting essentially of cells co-expressing P63 and KRT5 has a pouch-like shape. In one example, the cell layer overlays in an outward direction relative to the cell layer comprising or consisting essentially of cells co-expressing P63 and UPK1B and/or UPK2. In the example, the cells coexpressing P63 and KRT5 are localized in an outward direction relative to the cell layer comprising or consisting essentially of cells co-expressing P63 and UPK1B and/or UPK2. In one example, the cell layer comprising or consisting essentially of cells co-expressing P63 and KRT5 exists between the cell layer comprising or consisting essentially of cells co-expressing P63 and UPK1B and/or UPK2 and the cell layer comprising or consisting essentially of stromal-like cells described below.

In one example, the cell layer includes not less than 70%, not less than 75%, not less than 80%, preferably not less than 85%, and more preferably not less than 90% of stromal-like cells. In one example, the cell layer consists essentially of stromal-like cells. In one example, the cell layer comprising or consisting essentially of stromal-like cells has a pouch-like shape. In one example, the cell layer overlays in an outward direction relative to the cell layer comprising or consisting essentially of cells co-expressing P63 and KRT5. In the example, stromal-like cells are localized in an outward direction relative to the cell layer comprising or consisting essentially of cells co-expressing P63 and KRT5. In one example, the cell layer comprising or consisting essentially of stromal-like cells exists between the cell layer comprising or consisting essentially of cells co-expressing P63 and KRT5 and the cell layer comprising or consisting essentially of smooth muscle cells described below.

In one example, the cell layer includes not less than 70%, not less than 75%, not less than 80%, preferably not less than 85%, and more preferably not less than 90% of smooth muscle cells. In one example, the cell layer consists essentially of smooth muscle cells. In one example, the cell layer comprising or consisting essentially of smooth muscle cells has a pouch-like shape. In one example, the cell layer overlays in an outward direction relative to the cell layer comprising or consisting essentially of stromal-like cells. In the example, the smooth muscle cells are localized in an outward direction relative to the cell layer comprising or consisting essentially of stromal-like cells.

The term "stromal-like cell" herein refers to a cell that forms the supporting tissue of epithelial cells. Stromal-like cells include, for example, fibroblasts. In one embodiment, the cell layer comprising stromal-like cells predominantly includes vimentin (VIM)-expressing cells. In one embodiment, the cell layer comprising stromal-like cells is a cell layer localized, in the bladder organoid, between the cell layer comprising cells co-expressing P63 and KRT5 and the cell layer comprising smooth muscle cells expressing αSMA. The cells predominantly included in the cell layer express VIM.

The term "VIM" herein refers to intermediate filament specific to mesenchymal cells. VIM can be used as a mesenchymal stem cell marker. VIM expressed in cells or organoids can be detected or measured, for example, by immunostaining with anti-VIM antibody (e.g., Chicken Anti-Vimentin (NOVUS, #NB300-223)).

The term "smooth muscle cell" herein refers to an elongated, spindle-shaped mononuclear cell comprising numerous actin filaments and a few myosin filaments. In one embodiment, the cell layer comprising smooth muscle cells predominantly comprises αSMA-expressing cells. In one embodiment, the cell layer comprising smooth muscle cells predominantly comprises cells co-expressing αSMA and VIM.

The term "α-smooth muscle actin (αSMA)" herein refers to a protein belonging to the actin family and is also called ACTA2. αSMA can be used as a marker for smooth muscle cells. αSMA expressed in cells or organoids can be detected or measured, for example, by immunostaining with anti-αSMA antibody (Rabbit Anti-αSMA (Cell signaling, #19245T)).

Bladder organoids can be produced by a method comprising Process A: culturing pluripotent stem cells with the inducer medium A to induce differentiation into definitive endoderm cells, Process B: culturing the definitive endoderm cells with the inducer medium B, and then culturing them in the presence of extracellular matrix with an inducer medium B to form ventral hindguts, and Process c1: culturing them in the presence of extracellular matrix with an inducer medium C containing retinoic acid, fibroblast growth factor, and bone morphogenetic protein; Process c2: inducing them into a kidney or bladder or its peripheral area of a human or a non-human mammal; or Process c3: culturing them in the presence of mesenchymal stem cells or mesenchymal cells.

### Process c1: Induction of bladder epithelial differentiation (in vitro culture)

Process c1 comprises culturing a ventral hindgut organoid in the presence of extracellular matrix with an inducer medium C.

An "inducer medium C" comprises a basal medium and addition agents comprising retinoic acid, fibroblast growth factor, and bone morphogenetic protein. For example, the inducer medium C can be prepared by adding the addition agents (solid or liquid) to the basal medium (liquid). Concentrations of the addition agents added to the inducer medium C are appropriately adjusted by a person skilled in the art, considering the animal species which provide cells used for the culturing. The explanation of the basal medium for inducer medium A appropriately applies to the basal medium for inducer medium C. The inducer medium C may further contain heparin, protein-free medium (e.g., PFHM-II), antibiotics (e.g., penicillin/streptomycin, gentamicin), or antibiotic-antifungal mixture (e.g., Antibiotic-Antimycotic), or a combination thereof.

The inducer medium C may contain FGF (preferably FGF7) at 10-500 ng/ml, 30-250 ng/ml, or 50-150 ng/ml. The inducer medium C may contain FGF7 at 10-500 ng/ml, 30-250 ng/ml, or 50-150 ng/ml, or FGF4 at 20-1000 ng/ml, 60-500 ng/ml, or 100-300 ng/ml. The inducer medium C may contain FGF at a concentration exerting a fibroblast proliferation effect to the same degree as the effect exerted by FGF7 at 10-500 ng/ml, 30-250 ng/ml, or 50-150 ng/ml.

The inducer medium C may contain bone morphogenetic protein (preferably BMP4) at 3-150 ng/ml, 9-75 ng/ml, or 15-45 ng/ml. The inducer medium C may contain BMP at a concentration exerting an induction effect of alkaline phosphatase production to the same degree as the effect exerted by BMP4 at 3-150 ng/ml, 9-75 ng/ml, or 15-45 ng/ml. The inducer medium C may contain BMP at a concentration expressing a ventral hindgut marker (e.g., GATA3, P63, or HOXA13) to the same degree as the expression in the organoid cultured with the inducer medium C containing BMP4 at 3-150 ng/ml, 9-75 ng/ml, or 15-45 ng/ml. In this context, the organoid cultured with the inducer medium C may be a bladder organoid formed in Process b2 or b3 according to the present disclosure. The expression of the ventral hindgut marker can be measured, for example, by immunostaining methods. In the immunostaining method, the expression of the ventral hindgut marker can be measured with antibodies for each marker described in the present disclosure. In an embodiment, the inducer medium C contains BMP at a concentration exerting an induction effect of alkaline phosphatase production to the same degree as the effect by BMP4 at 3-150 ng/ml, 9-75 ng/ml, or 15-45 ng/ml.

The inducer medium C may contain retinoic acid (preferably all-trans retinoic acid) at 0-1 µM, 10 nM-500 nM, 30 nM-300 nM, or 50 nM-150 nM.

The explanation for the culturing of spheroids of the definitive endoderm cells in the presence of extracellular matrix with the inducer medium b1 described above appropriately applies to the culturing of ventral hindgut organoids in the presence of extracellular matrix with the inducer medium C. For example, the culturing of ventral hindgut organoids in the presence of extracellular matrix with the inducer medium C can be conducted under known cell culture conditions. The known cell culture conditions may be to keep at 37°C under 5% CO₂. The temperature for culturing is not limited to 37°C but may appropriately use any temperature known in the field of cell culture. The CO₂ concentration is not limited to 5% but may appropriately use any known concentration of CO₂ in the field of cell culture. The culture of the ventral hindgut organoid can be carried out for 5-30 days, 5-25 days, 5-20 days, 5-15 days, or 5-10 days.

The form of extracellular matrix (gel form or form of dispersing component) in Process c1 may be the same as or different from each in Process b1 and Process b2. In an embodiment, the form of the extracellular matrix in Process c1 is the same as that of each extracellular matrix in Process b1 and Process b2. In an embodiment, an extracellular matrix gel including ventral hindgut organoids may be the extracellular matrix gel formed in Process b1 and Process b2 or an extracellular matrix gel newly formed by collecting the ventral hindgut organoids from the extracellular matrix gel after Process b2 and forming it in which the collected ventral hindgut organoids are included. For the simplicity of operation, the extracellular matrix gel including the ventral hindgut organoid in Process c1 is the extracellular matrix gel formed in Process b1 and Process b2.

In an embodiment, Process c1 comprises culturing ventral hindgut organoids in extracellular matrix gels with the inducer medium C to form bladder organoids. In an embodiment, Process c1 comprises culturing the ventral hindgut organoids on an extracellular matrix gel with the inducer medium C to form bladder organoids. In an embodiment, Process c1 comprises culturing the ventral hindgut organoids in the presence of extracellular matrix as a dispersing component with the inducer medium C to form bladder organoids.

In an embodiment, the culturing of ventral hindgut organoids with the inducer medium C comprises culturing the ventral hindgut organoids with an inducer medium C, STEMdiff APEL2 medium (10 or 30 ng/ml BMP4, 0.1-1 µM All-trans retinoic acid, 100 ng/ml FGF7 or FGF9, 1 µg/mL Heparin, 2% PFHM-II, Antibiotic-Antimycotic) for at least six days to induce bladder epithelium differentiation. Add the inducer medium C onto the top of the Cell Culture Insert gel at 200-400 µL and under it at 300-600 µL and exchange the medium every two days. Preferably keep the culture until the expressions of the bladder epithelial markers UPK2, P63, and KRT5 are detected. Then, add PPAKγ agonist (0.1-10 µM Rosiglitazone) to the inducer medium C to mature the bladder epithelial cells.

In an embodiment, the bladder organoids can be produced by a method comprising Process A: culturing pluripotent stem cells with the inducer medium A to induce differentiation into definitive endoderm cells, Process b1: culturing the definitive endoderm cells with an inducer medium B containing fibroblast growth factor and GSK3β inhibitor (inducer medium b), and then culturing them in the presence of extracellular matrix (preferably in an extracellular matrix gel) with the inducer medium B (inducer medium b1) to form hindgut organoids, and Process b2: culturing the hindgut organoids in the presence of extracellular matrix (preferably in an extracellular matrix gel) with an inducer medium B containing fibroblast growth factor, GSK3β inhibitor, and bone morphogenetic protein (inducer medium b2) to form ventral hindgut organoids, and Process c1: culturing the ventral hindgut organoids in the presence of extracellular matrix with an inducer medium C containing retinoic acid, fibroblast growth factor, and bone morphogenetic protein.

In one embodiment, the bladder organoids can be produced by a method comprising Process A: culturing pluripotent stem cells with the inducer medium A to induce differentiation into definitive endoderm cells,
Process b3: culturing the definitive endoderm cells with an inducer medium B containing fibroblast growth factor and GSK3β inhibitor, and optionally further containing bone morphogenetic protein (inducer medium b) and then culturing them in the presence of extracellular matrix (preferably, extracellular matrix as a dispersing component) with an inducer medium B containing fibroblast growth factor, GSK3β inhibitor, and bone morphogenetic protein (inducer medium b3) to form ventral hindgut organoids, and Process c1: culturing the ventral hindgut organoids in the presence of extracellular matrix with an inducer medium C containing retinoic acid, fibroblast growth factor, and bone morphogenetic protein.

### Process c2: Introduction into a kidney or bladder or its peripheral area of a mammals

Process c2 comprises introducing a ventral hindgut organoid into a kidney or bladder or its peripheral area of a human or a non-human mammal to form a bladder organoid. In an embodiment, Process c2 comprises introducing the ventral hindgut organoid into the kidney or bladder or its peripheral area of a human or a non-human mammal to form a bladder organoid. When the ventral hindgut organoid exists in an extracellular matrix gel, Process c2 may further comprise collecting the ventral hindgut organoid from the extracellular matrix gel before introducing it into a human or non-human mammal.

The term "non-human mammal" herein may be, for example, a rodent such as a mouse, rat, guinea pig, hamster; a non-human primate such as a chimpanzee; artiodactyl such as cattle, goat, sheep; perissodactyl such as a horse; a companion animal such as rabbit, dog, cat. In an embodiment, the non-human mammal is a rodent or non-human primate.

The term "kidney" herein refers to an organ in the urinary system that produces urine by filtering and draining waste products or excess water from the blood. The kidney may be herein a normal kidney without specific damage or disease, a damaged kidney, or the kidney suffering from renal disease. In an embodiment, the kidney is a normal kidney. In an embodiment, the kidney is a damaged kidney or the kidney suffering from renal disease.

The term "bladder" herein refers to a pouch-like organ that temporarily stores urine delivered from the kidneys. The bladder may be a normal bladder without specific damage or disease, a damaged bladder, or a bladder suffering from bladder disease. In an embodiment, the bladder is a normal bladder. In an embodiment, the bladder is a damaged bladder or a bladder suffering from bladder disease.

A "peripheral area" of the kidney or bladder refers to a tissue or area contiguous to or vicinity of the urinary system. The urinary system includes the kidneys, ureters, bladder, and urethra. The peripheral area of the kidney or bladder may be, for example, intraperitoneal or mesenteric.

"Introduction" of a ventral hindgut organoid into a kidney or bladder or its peripheral area refers to manipulation to place the ventral hindgut organoid in the kidney or the bladder or its peripheral area. Introduction of the ventral hindgut organoid into the kidney or bladder or its peripheral area comprises, for example, transplanting the ventral hindgut organoid in an extracellular matrix gel into the kidney or bladder or its peripheral area by surgical technique. Introduction of the ventral hindgut organoid into the kidney or bladder or its peripheral area comprises, for example, injecting the ventral hindgut organoid in solution into the kidney or bladder or its peripheral area with an instrument such as a syringe.

For example, the ventral hindgut organoid may be produced from a pluripotent stem cell originating from a different animal species than the human or the non-human mammal into which it is introduced into a kidney or bladder or its periphery area or originating from the same animal species or the same individual. In an embodiment, the method for producing a bladder organoid comprises introducing the ventral hindgut organoid produced from human ES cells or human iPS cells into a kidney or bladder or its peripheral area of a human or a non-human mammal.

In an embodiment, introducing a ventral hindgut organoid into the kidney comprises implanting the ventral hindgut spheroid under the renal capsuleof a NOD SCID mouse. The transplanted ventral hindgut organoid is cultured *in vivo* for 4 weeks by keeping the transplanted mice for 4 weeks. The *in vivo* culture can form a bladder organoid having a pouch-like structure. In an embodiment, *in vivo* culture can be carried out for 1-6 weeks, 2-5 weeks, or 3-5 weeks.

In an embodiment, the bladder organoid can be produced by the method comprising Process A: culturing pluripotent stem cells with the inducer medium A to induce differentiation into definitive endoderm cells, Process b1: culturing the definitive endoderm cells with an inducer medium B containing fibroblast growth factor and GSK3β inhibitor (inducer medium b), and then culturing them in the presence of extracellular matrix (preferably in an extracellular matrix gel) with the inducer medium B (inducer medium b1) to form hindgut organoids, and Process b2: culturing the hindgut organoids in the presence of extracellular matrix (preferably in an extracellular matrix gel) with an inducer medium B containing fibroblast growth factor, GSK3β inhibitor, and bone morphogenetic protein (inducer medium b2) to form ventral hindgut organoids, and Process c2: introduction them into a kidney or bladder or its peripheral area of a human or a non-human mammal.

In an embodiment, the bladder organoid can be produced by the method comprising Process A: culturing pluripotent stem cells with the inducer medium A to induce differentiation into definitive endoderm cells, Process b3: culturing the definitive endoderm cells with an inducer medium B containing fibroblast growth factor and GSK3β inhibitor, and optionally further containing bone morphogenetic protein (inducer medium b), and then culturing them in the presence of extracellular matrix (preferably, extracellular matrix as dispersing component) with an inducer medium B containing fibroblast growth factor, GSK3β inhibitor, and bone morphogenetic protein (inducer medium b3) to form ventral hindgut organoids, and Process c2: introduction them into a kidney or bladder or its peripheral area of a human or a non-human mammal.

### Process c3: Co-culture in the presence of mesenchymal stem cells or mesenchymal cells

Process c3 comprises culturing the ventral hindgut organoid in the presence of mesenchymal stem cells or mesenchymal cells to form a bladder organoid. When the ventral hindgut organoid exists in an extracellular matrix gel, Process c3 may further comprise collecting the ventral hindgut organoid from the extracellular matrix gel before the co-culture.

The term "mesenchymal cell" herein refers to a cell originating from mesenchyme at the fetal stage of a multicellular organism. For example, mesenchymal cells can differentiate into supporting tissues, connective tissues, osteocytes, cartilage cells, and adipose cells. In an embodiment, the mesenchymal cells are bladder mesenchymal cells. Bladder mesenchymal cells can be prepared, for example, from non-human mammals according to known methods or the methods described in Examples herein. For example, mesenchymal cells prepared from non-human mammals comprise embryonic fibroblasts. In an embodiment, the embryonic fibroblasts are mouse embryonic fibroblasts (MEFs). For example, mesenchymal cells can be prepared by inducing differentiation from ES cells or iPS cells according to known methods. For example, mesenchymal cells may be commercially obtained.

The term "mesenchymal stem cell" herein refers to a cell capable of selfreplicating and differentiating into non-epithelial cells such as connective tissue, bone cells, cartilage cells, and adipose cells, which form mesenchyme. For example, mesenchymal stem cells can be prepared from ES cells or iPS cells by inducing differentiation according to known methods. For example, mesenchymal stem cells may be commercially obtained. For example, mesenchymal stem cells can be collected from a living organism according to known methods. Mesenchymal stem cells are known to exist in a living body, for example, in dental pulp or bone marrow fluid.

In an embodiment, Process c3 comprises culturing the ventral hindgut organoid in the presence of mesenchymal stem cells or mesenchymal cells with a medium to form a bladder organoid. The medium used in Process c3 may be, for example, a basal medium, which optionally contains addition agents according to the present disclosure. In an embodiment, the medium is characterized by adding no extracellular matrix. In an embodiment, the medium is STEMdiff APEL2 medium (STEMCELL Tecnologies) (supplemented with 2% PFHM-II and Antibiotic-Antimycotic) or STEMdiff APEL2 medium (STEMCELL Tecnologies) (supplemented with 2% PFHM-II, 2% FBS, and Antibiotic-Antimycotic).

In an embodiment, Process c3 comprises stationary culture of ventral hindgut organoids in the presence of mesenchymal stem cells or mesenchymal cells, followed by rotary suspension culture of the ventral hindgut organoids and mesenchymal stem cells or mesenchymal cells to form bladder organoids. For example, the rotary suspension culture can be carried out with a three-dimensional rotary suspension culture device, CellPet 3D-iP (JTEC Corporation). The rotation speed at the rotary suspension culture may be set by a person skilled in the art so that cell aggregates do not settle by gravity and touch the culture vessel. The rotational speed may be, for example, 1-50 rpm, 1-30 rpm, 1-15 rpm, 1-10, 2-50 rpm, 2-30 rpm, 2-15 rpm, 2-10, 3-50 rpm, 3-30 rpm, 3-15 rpm or 3-10 rpm.

In an embodiment, Process c3 comprises rotary suspension culture of the ventral hindgut organoid or bladder organoid together with E12.5 mouse bladder mesenchymal cells for more than two weeks to form bladder organoids, including bladder epithelial cells, stromal-like cells, and smooth muscle cells, indicating a pouch-like structure. For example, the culture in Process c3 can be carried out for 1-6 weeks, 2-5 weeks, or 3-5 weeks.

In an embodiment, the bladder organoid can be produced by the method comprising Process A: culturing pluripotent stem cells with the inducer medium A to induce differentiation into definitive endoderm cells, Process b1: culturing the definitive endoderm cells with an inducer medium B containing fibroblast growth factor and GSK3β inhibitor (inducer medium b) and then culturing them in the presence of extracellular matrix (preferably in an extracellular matrix gel) with the inducer medium B (inducer medium b1) to form hindgut organoids, and Process b2: culturing the hindgut organoids in the presence of extracellular matrix (preferably in an extracellular matrix gel) with an inducer medium B containing fibroblast growth factor, GSK3β inhibitor, and bone morphogenetic protein (inducer medium b2) to form ventral hindgut organoids, and Process c3: culturing them in the presence of mesenchymal stem cells or mesenchymal cells.

In an embodiment, the bladder organoid can be produced by the method comprising Process A: culturing pluripotent stem cells with the inducer medium A to induce differentiation into definitive endoderm cells, Process b3: culturing the definitive endoderm cells with an inducer medium B containing fibroblast growth factor and GSK3β inhibitor and optionally further containing bone morphogenetic protein (inducer medium b), and then culturing them in the presence of extracellular matrix (preferably, extracellular matrix as a dispersing component) with an inducer medium B containing fibroblast growth factor, GSK3β inhibitor, an bone morphogenetic protein (inducer medium b3) to form ventral hindgut organoids, and Process c3: culturing them in the presence of mesenchymal stem cells or mesenchymal cells.

The explanation of each term for each process in the induction of differentiation into definitive endoderm cells (Process A), the formation of ventral hindgut organoid (Process B), and the formation of bladder organoid (Process C), as well as the explanation of embodiments including culture conditions appropriately apply to the corresponding term and process in their embodiments, respectively.

### [Non-human mammal comprising ventral hindgut organoid or bladder organoid, or method for producing the same]

An aspect of the present disclosure provides a method for producing a non-human mammal comprising a ventral hindgut organoid or bladder organoid in a kidney or bladder thereof or its peripheral area. Another aspect of the present disclosure provides a non-human mammal comprising a ventral hindgut organoid or bladder organoid in a kidney or bladder thereof, or its peripheral area.

A non-human mammal comprising a ventral hindgut organoid in a kidney or bladder thereof or its peripheral area can be produced by a method comprising introducing a ventral hindgut organoid according to the present disclosure into a kidney or bladder or its peripheral area of a non-human mammal. The non-human mammal comprising a bladder organoid in the kidney or bladder or its peripheral area can be produced by a method comprising introducing a ventral hindgut organoid according to the present disclosure into the kidney or bladder or its peripheral area of a non-human mammal and keeping the non-human mammal into which the ventral hindgut organoid according to the present disclosure is introduced. Keeping the non-human mammals comprises, for example, feeding known diets for the non-human mammal.

In one example, a non-human mammal comprising a ventral hindgut organoid, as the kidney cancer model or a bladder cancer model, in the kidney or bladder or its peripheral area can be produced by introducing tumor cells or tumor fragments into a ventral hindgut organoid according to the present disclosure and introducing the ventral hindgut organoid including the tumor cells or tumor fragments into the kidney or bladder or its peripheral area of a non-human mammal. The non-human mammal comprising a ventral hindgut organoid, the kidney cancer model or a bladder cancer model, in the kidney or bladder or its peripheral can be used in a method for assessing drug responsiveness of a candidate substance for treating kidney cancer or bladder cancer, the method comprising contacting the non-human mammal with a candidate substance for treating kidney cancer and assessing the effect by the candidate substance.

### [Method for assessing drug responsiveness to test substance]

A method for assessing drug responsiveness to a test substance, comprising contacting the test substance with a ventral hindgut organoid, a bladder organoid, or a non-human mammal comprising a ventral hindgut organoid or bladder organoid according to the present disclosure and measuring drug responsiveness to the test substance in the ventral hindgut organoid, the bladder organoid, or the non-human mammal.

The term "test substance" herein may be, for example, a small molecule compound, protein (e.g., antibody), DNA, RNA, small interfering RNA, or antisense oligonucleotide. The test substance may be, for example, a drug for treating kidney or bladder disorders or diseases, kidney or bladder cancer, or a candidate substance thereof. For example, the test substance may be one or a mixture of two or more test substances. The test substance is preferably one substance.

"Contacting" a test substance with a ventral hindgut organoid, a bladder organoid, or a non-human mammal refers to placing the test substance and the ventral hindgut organoid, bladder organoid, or non-human mammal in a situation where they can come into contact. Contacting a test substance with a ventral hindgut organoid, a bladder organoid, or a non-human mammal may be, for example, mixing the test substance with a culture medium including the ventral hindgut organoid or bladder organoid. Contacting a test substance with a non-human mammal comprising a ventral hindgut organoid or bladder organoid in a kidney or bladder thereof or its peripheral area may be, for example, by orally or parenterally administrating the test substance to the non-human mammal.

Drug responsiveness comprises, for example, structural or functional changes in the ventral hindgut organoid caused by the test substance. Drug responsiveness comprises, for example, changes in the concentration of the test substance by the ventral hindgut organoid.

### [Regenerative medicine composition or method for producing the same]

An aspect of the present disclosure provides a regenerative medicine composition for treating bladder injury or bladder disease.

The term "regenerative medicine composition" herein comprises a ventral hindgut organoid or bladder organoid according to the present disclosure. The regenerative medicine compositions according to the present disclosure can be used to treat bladder injuries or bladder diseases in mammals.
For example, the regenerative medicine composition may appropriately comprise a pharmaceutically acceptable carrier. The term "pharmaceutically acceptable carrier" herein refers to any component, which is safe in mammals and has low allergic reactivity, other than the ventral hindgut organoid or bladder organoid according to the present disclosure. The pharmaceutically acceptable carrier includes, for example, aqueous or non-aqueous solvent, solution (e.g., saline, basal medium, or cell suspension solution), cryoprotectant (e.g., glycerol), water-soluble polymer (e.g., dextran), or buffer (e.g., phosphate buffer), which are suitable for pharmaceutical administration. The regenerative medicine composition can be appropriately produced according to known methods. In one example, the regenerative medicine composition according to the present disclosure can be produced by mixing a ventral hindgut organoid or bladder organoid according to the present disclosure with the pharmaceutically acceptable carrier (e.g., basal medium).

The regenerative medicine composition is administered to a mammal in need thereof, for example, by surgical implantation into a predetermined site in the bladder or by injection into a predetermined site in the bladder with a syringe or other device. A mammal to which the regenerative medicine composition is administered is preferably the same species and more preferably the same individual as the mammal providing pluripotent stem cells used to produce the ventral hindgut organoid or bladder organoid from the viewpoint of reducing graft rejection.

The term "mammal" in connection with the regenerative medicine composition refers to, for example, a human and non-human mammal. The non-human mammal may be, for example, a rodent such as a mouse, rat, guinea pig, hamster; a non-human primate such as a chimpanzee; artiodactyl such as cattle, goat, sheep; perissodactyl such as a horse; a companion animal such as rabbit, dog, and cat. In an embodiment, the mammal is human.

The term "bladder injury" or "bladder disease" herein may be, for example, a bladder damaged by trauma, radiation cystitis, bladder damaged by diabetes, ischemia, and the like, bladder damaged by a drug harmful to bladder tissue, cystitis, or bladder cancer.

The regenerative medicine composition according to the present disclosure can be used in a method for treating bladder injury or disease in a mammal. An embodiment provides a method for treating bladder injury or disease, the method comprising administering a regenerative medicine composition comprising a ventral hindgut organoid or bladder organoid according to the present disclosure to a mammal in need thereof.

### [Method for treating bladder injury or disease]

An aspect of the present disclosure provides a method for treating bladder injury or disease in a mammal. The method for treating a bladder injury or disease in a mammal comprises inducing the ventral hindgut organoid, bladder organoid, or regenerative medicine according to the present disclosure into a kidney or bladder or its peripheral area of the mammal in need thereof. In an embodiment, the method for treating bladder injury or disease in a mammal comprises introducing the regenerative medicine composition according to the present disclosure into a kidney or bladder or its peripheral area of the mammal in need thereof.

The term "mammal in need thereof" herein refers to a mammal having or suspected of having bladder injury or disease. The mammal having bladder injury or disease refers to a mammal diagnosed as having bladder injury or disease by a medical worker (e.g., physician) according to prescribed diagnostic criteria. The mammal suspected of having bladder injury or disease may be, for example, a mammal suspected of having bladder injury or disease based on the mammal's action history (e.g., received or is receiving trauma, radiation therapy, and drug harmful to bladder tissue) or medical history (e.g., suffering or suffered from diabetes, ischemia, cystitis, or bladder cancer). The mammal according to the aspect is preferably a human or a non-human primate, more preferably a human.

### "Treatment" of bladder injury or disease comprises the maintenance, reduction, or disappearance of its symptoms or condition. Treatment of bladder injury or disease comprises curing the bladder injury or disease.

The species of pluripotent stem cells used to produce the ventral hindgut organoid, bladder organoid, or regenerative medicine composition to be introduced into the mammal in need thereof and the mammal in need should be the same species or the same individual from the viewpoint of reducing graft rejection. The mammal in need thereof is preferably the same species and more preferably the same individual as the mammal providing pluripotent stem cells used to produce the ventral hindgut organoid, bladder organoid, or regenerative medicine composition from the viewpoint of reducing graft rejection.

The term "comprising" herein means inclusive of recited elements and/or steps and other additional elements and/or steps. The term "consisting of" herein means inclusive of recited elements and/or steps but exclusive of other additional elements and/or steps. The term "consisting essentially of" herein means inclusive recited elements and/or steps and inclusive other additional elements and/or steps to the extent that they do not affect the novel technical features of the cell layer, organoids, compositions, and methods. The term "not substantially comprising" herein does not exclude "completely excluding.

Unless described otherwise, the terms and explanations for embodiments provided by the present disclosure appropriately apply to their aspects and embodiments provided by present disclosure.

Specific examples are described below, but they represent preferred embodiments of the invention and are not intended to limit in any way the invention as recited in the Claims.

### EXAMPLES

### Materials and Methods

The following growth factors and compounds were prepared:
Recombinant Human/Mouse/Rat Activin A (R&D SYSTEMS, #338-AC), Recombinant Human FGF4 (R&D SYSTEMS, #7460-F4), Recombinant Human BMP4 (R&D SYSTEMS, #314-BP), Recombinant Human KGF/FGF7 (R&D SYSTEMS, #251-KG), CHIR99021 (TOCRIS, #4423), All-trans retinoic acid (SIGMA, R2625).

### Immunofluorescence staining

To prepare frozen sections, bladder organoids or grafts were incubated overnight in 4% PFA at 4°C with shaking and then were washed three times with PBS(-) and replaced in sucrose solution. Sucrose solutions of 10%, 20%, and 30% were prepared in PBS(-) and incubated at 4°C until samples settled at each step. Next, frozen blocks of the bladder organoids were prepared by removing Matrigel surrounding the organoids with tweezers and embedding them in OCT Compound. Sections of 10 µm were prepared and incubated in PBS(-) supplemented with 10% Donkey serum and 0.3% Triton-X (hereafter referred to as "Blocking Buffer") for 1 hour at room temperature for blocking. Next, they were incubated with a primary antibody diluted with Blocking Buffer overnight at 4°C. Then they were washed three times with PBS(-) and incubated with a secondary antibody diluted with PBS(-) overnight at 4°C. Nuclei were stained with DAPI. The nuclei were then washed three times with PBS(-), embedded in FluorSave Reagent (Millipore, #345789), and observed under a confocal microscope (ZEISS, LSM800).

### [Example 1] Production of Ventral Hindgut Organoids

Figure 1 is a flowchart diagram illustrating an overview of the production of bladder organoids according to one embodiment. Figure 1 shows Process A for inducing differentiation of human iPS cells into definitive endoderm cells, Process b1 for inducing differentiation of the definitive endoderm cells into hindgut organoids, Process b2 for inducing differentiation of the hindgut organoids into ventral hindgut organoids, and Process c1 for inducing differentiation of the ventral hindgut organoids into bladder-like organoids. Process B, including Process b1 and Process b2, induces differentiation into the ventral hindgut. Example 1 produces ventral hindgut organoids by performing Process A, Process b1, and Process b2.

### Pre-culture of human iPS cells

Human iPS cells (1502.3 strain, given by Dr.Melissa Little, Murdoch Children's Research Institute) were maintained and cultured in StemFit AK02N medium (REPROCELL) on a culture plate surface coated with iMatrix-511 (nippi). The cells were peeled by TrypLE Select (Thermo Fisher Scientific) to obtain a cell suspension. The cell suspension was centrifuged at 200 rcf for 5 min at room temperature, the supernatant was removed, and the precipitate was resuspended in StemFit AK02N (10 µM Y-27632). To the suspension, iMatrix-511 (nippi) was added at 0.25 µg/cm2, and the cells were seeded at 60,000 cells/cm² onto a 6-well plate (Corning) and incubated at 37°C under 5% CO₂ for one day.

### Process A: Induction of differentiation into definitive endoderm cells

The culture medium was exchanged for an inducer medium A, STEMdiff APEL2 medium (STEMCELL Tecnologies) supplemented with 100 ng/ml ActivinA, 1µM CHIR99021, 2% PFHM-II, and Antibiotic-Antimycotic, and the cells were cultured for three days to induce differentiation into definitive endoderm cells. The medium was exchanged daily after day 2. Human iPS cells assembled into colonies on day 1 after the induction of differentiation but began to expand outside the colonies on day 2 after the induction of differentiation. On day 3 after the induction of differentiation, the cells proliferated in a sheet-like shape and reached 100% confluent. Cells in a paving-stone arrangement, typical of embryonic endoderm, were induced.

### Process b1: Induction of hindgut differentiation

After induction of embryonic endoderm (on day 3 after the induction of differentiation), the inducer medium A was exchanged for an inducer medium b1, STEMdiff APEL2 medium (supplemented with 200 ng/ml FGF4, 8µM CHIR99021, 1µg/mL Heparin, 2% PFHM II, and Antibiotic-Antimycotic), and the differentiation-induced definitive endoderm cells were cultured for four days.

On day 7 after the induction of differentiation, floating spheroids were collected in 35 mm dishes. Next, the remaining spheroids in the wells from which the floating spheroids were collected were floated by pipetting a medium over the spheroids and collected in the same 35 mm dish.

Matrigel Growth Factor Reduced (Corning) (protein concentration at 10mg/mL) was diluted to 50% concentration in an inducer medium b1, added to Cell Culture Insert Transparent PET Membrane 24 well 8.0µm pore-size (corning) at 50 µL/well, and incubated at 37°C for 30 min to gel. Then, the collected spheroids of 40-50 were added to 50% Matrigel solution of 50 µL and mixed. All the mixture was added onto the gelled 50% Matrigel and incubated at 37°C under 5% CO₂ for 30 min to gel and obtain a Cell Culture Insert gel. The inducer medium b1 was added onto the top of the Cell Culture Insert gel at 200 µL and under the gel at 300 µL, and the gel was incubated at 37°C under 5% CO₂ for four days. The medium was exchanged daily in 2D culturing and every two days in 3D culturing. The spheroids in the 50% Matrigel grew by the culture in the inducer medium b1 and formed slightly rounded tubular cellular structures (hindgut organoids).

### Process b2: Ventralization of hindgut

After induction of hindgut differentiation (on day 11 after the induction of differentiation), the inducer medium b1 was exchanged for an inducer medium b2, STEMdiff APEL2 medium (supplemented with 30 ng/ml BMP4, 200 ng/ml FGF4, 8µM CHIR99021, 1µg/mL Heparin, 2% PFHM-II, and Antibiotic-Antimycotic), and the hindgut induced in the Cell Culture Insert gel was cultured for three days to ventralize the hindgut. The inducer medium b2 was added onto the top of the Cell Culture Insert gel at 200 µL and under the gel at 300 µL, and the medium was exchanged every two days. When cultured in the hindgut-ventralization medium, the hindgut organoids grew slowly and became spherical cellular structures (ventral hindgut organoids).

The ventral hindgut organoids (on day 14 of the induction of differentiation) were immunofluorescently stained with the antibodies listed in the following table.

**[Table 1]**

| Antibody | Catalog No. | Supplier |
|---|---|---|
| Anti-P63 antibody rabbit monoclonal (EPR5701) | ab124762 | Abcam |
| Anti-CDX2 antibody mouse monoclonal (CX2-88) | MU392A-UC | BioGeneX |
| Anti-SOX2 antibody goat polyclonal | AF2018 | R&D systems |
| Anti-Cytokeratin8 antibody rat monoclonal (TROMA-1) | TOROMA-1 | Developmental Studies Hybridoma Bank |
| Alexa Fluor 488 Donkey Anti-Mouse IgG | A21202 | Life Technologies |
| Alexa Fluor 568 Donkey Anti-Rabbit IgG | A10042 | Life Technologies |
| Alexa Fluor 647 Donkey Anti-Goat IgG | A21447 | Life Technologies |
| Alexa Fluor 488 Donkey Anti-rat IgG | A21208 | Life Technologies |

The ventral hindgut organoids expressed a ventral hindgut marker P63, weakly expressed or not expressed dorsal hindgut markers CDX2 and SOX2, respectively, and expressed an intestinal epithelial marker KRT8. RT-PCR also indicated that the transcription of HOXA13, a marker of posterior intestinal tract marker and Cloaca region, increased. These results are summarized in the following table.

**[Table 2]**

| | Early hindgut organoids | Hindgut organoids | Ventral hindgut organoids |
|---|---|---|---|
| Days after induction of differentiation | 9 days | 11 days | 14 days |
| P63 | + | ++ | +++ |
| CDX2 | +++ | +++ | + |
| SOX2 | ++ | + | - |
| KRT8 | ++ | +++ | ++ |
| HOXA13 | - | - | ++ |

| | | | |
|---|---|---|---|
| +++: Strongly expressed ++: Strongly expressed in some cells or expressed +: Expressed in some cells or weakly expressed -: No increase in expression or no expression | | | |

### [Comparative Example 1]

### Induction of differentiation into intestine-like organoids (SATB2 positive)

In Process b2 of Example 1, the hindgut organoids in the Cell Culture Insert gel obtained in Process b1 were cultured in STEMdiff APEL2 medium (supplemented with 100 ng/ml BMP2, 100 ng/ml EGF, 500 ng/ml RSPO1, and 0.1µM retinoic acid) for 21 days to induce differentiation of intestine-like organoids (SATB2-positive).

The intestine-like organoids expressed a colon or rectum marker SATB2 (Fig. 2a). The intestine-like organoids expressed a mid/hindgut marker CDX2 in part and expressed an epithelial tissue marker ECAD (Figs. 2b and 2c). Fluorescent images of SATB2, CDX2, ECAD, and a nuclear marker DAPI (Fig. 2d) showed that specific cell types did not form a cell layer structure in which they were regularly localized.

The intestine-like organoids obtained in Comparative Example 1 also expressed a ventral hindgut/Cloaca marker P63, a bladder epithelial marker UPK2, and a bladder epithelial marker KRT5. The results regarding P63, UPK2, and KRT5 also showed that the intestine-like organoids obtained in Comparative Example 1 did not have a cell layer structure in which specific cell types are regularly localized.

Comparative Example 1 used a combination of additional agents (BMP2, EGF, RSPO1, and retinoic acid) different from the combination of additional agents (BMP4, FGF4, and CHIR99021) used in Process b2 of Example 1. The cellular structures obtained in Comparative Example 1 did not have a cell layer structure in which specific cell types were regularly localized, like the ventral hindgut organoids obtained in Example 1.

Cellular structures, obtained by using other combinations of additional agents (a combination of EGF, RSPO1, and retinoic acid; a combination of EGF, RSPO1, retinoic acid, and Noggin; a combination of EGF, RSPO1, retinoic acid, Noggin, and FGF7; and a combination of EGF, RSPO1, retinoic acid, BMP2, and FGF7) different from the combination of additional agents (BMP4, FGF4, and CHIR99021) used in Process b2 of Example 1, did not have a cell layer structure in which specific cell types were regularly localized, like the ventral hindgut organoids obtained in Example 1, either.

### [Example 2] Production of Bladder-like Organoids

### Process c1: Induction of bladder epithelial differentiation

After ventralization of hindgut (on day 14 after the induction of differentiation), the inducer medium b2 was exchanged for an inducer medium C, STEMdiff APEL2 medium (supplemented with 30 ng/ml BMP4, 100nM All-trans retinoic acid, 100 ng/ml FGF7, 1µg/mL Heparin, 2% PFHM-II, and Antibiotic-Antimycotic). The spherical cellular structures were cultured for six days to induce bladder epithelial cells. The inducer medium C was added onto the top of the Cell Culture Insert gel at 200 µL and under the gel at 300 µL, and the medium was exchanged every two days. The induced bladder-like organoids had a spherical shape.

The bladder-like organoids were immunofluorescently stained. Example 2 and below-described Example 3 used the following antibodies for immunofluorescence staining. Primary antibodies used for immunofluorescence staining were as follows: Mouse Anti-Uroplakin II (1:100, BIOCARE MEDICAL, #ACR3051C), Rabbit Anti-P63 (1:100, Abcam, #ab124762), Chicken Anti-Keratin5 (1:300, BioLegend, #905903), Goat Anti-ECAD (1:300, R&D SYSTEMS, #AF648). Secondary antibodies used for immunofluorescence staining were as follows: Alexa Fluor 488 Donkey Anti-Mouse IgG (1:400, Life Technologies, #A21202), Alexa Fluor 568 Donkey Anti-Rabbit IgG (1:400, Life Technologies, #A10042), Alexa Fluor 647 Donkey Anti-Goat IgG (1:400, Life Technologies, #A21447), Alexa Fluor 647 Donkey Anti-Chicken IgY (1:400, Jackson ImmunoResearch, #703-606-155).

The bladder-like organoid expressed an epithelial tissue marker ECAD (Fig. 3e). The bladder-like organoid expressed bladder epithelial markers, UPK2 (Fig. 3a), P63 (Fig. 3b), and KRT5 (Fig. 3c). In Figures 3a and 3d, the signals observed at the outermost periphery of the bladder-like organoids are non-specific signals derived from Matrigel. Fig. 3d, a fluorescent overlay image of UPK2, P63, and KRT5, shows the bladder-like organoid in which basal cell-like cells co-expressing P63 and KRT5 are localized along the outermost periphery of the organoid, Intermediate cell-like cells co-expressing P63 and UPK2 are localized in an inward direction relative to a region where KRT5-basal cell-like cells are localized, and Superficial cell-like cells expressing UPK2 but not expressing P63 are localized in an inward direction relative to an area where the Intermediate cell-like cells are localized. A layer structure of these cell types in bladder-like organoids is similar to that of the developing bladder epithelium *in vivo* (Fig. 5g). Since Krt5-basal cells expressing KRT5 are a cell type that appears in the late stage of bladder development, the fact that the bladder-like organoid had a small number of KRT5-expressing cells suggests that the induced bladder-like organoid is in an immature stage.

### [Comparative Example 2]

The hindgut organoid was induced and differentiated into bladder epithelium in the same way as in Example 2, except for using the inducer mediums b2 and C from which BMP4 was eliminated. Cellular structures formed in Comparative Example 2 were immunofluorescently stained as in Example 2. The cellular structures formed in Comparative Example 2 expressed the bladder epithelial marker P63 (Fig. 4b) and the epithelial tissue marker ECAD (Fig. 4c), like the bladder-like organoids formed in Example 2. However, the cellular structures formed in Comparative Example 2 did not express the bladder epithelial marker UPK2 as observed in the bladder-like organoids formed in Example 2 (Fig. 3a and Fig. 4a). In Figure 4a, the signal observed at the outermost periphery of the bladder-like organoid is the non-specific signal derived from Matrigel. Thus, the cellular structures formed in Comparative Example 2 do not have the cell layer structure observed in the bladder-like organoids formed in Example 2.

### [Example 3] In vivo production of bladder organoids

### Pre-culture of human iPS cells

Human iPS cells were pre-cultured in the same way as in Example 1, except for seeding the cells at 90,000 cells/cm² onto a 6-well plate.

### Process A: Induction of differentiation into definitive endoderm cells

Human iPS cells were induced and differentiated into definitive endoderm cells in the same way as in Example 1.

### Process b1: Induction of hindgut differentiation

Induction of hindgut differentiation was conducted in the substantially same way as in Example 1, except that the Cell Culture Insert gel was made by diluting Matrigel Growth Factor Reduced (Corning) (protein concentration of 10 mg/mL) in an inducer medium b1 to a concentration of 2.5 mg/mL and gelating and contained 90-100 spheroids formed by 2-dimensionally culturing definitive endoderm cells.

### Process b2: ventralization of hindgut

After induction of hindgut differentiation (on day 11 after the induction of differentiation), the inducer medium b1 was exchanged for an inducer medium b2, STEMdiff APEL2 medium (supplemented with 30 ng/ml BMP4, 100 ng/ml FGF7, 200 ng/ml FGF4, 8µM CHIR99021, 1µg/mL Heparin, 2% PFHM-II, and Antibiotic-Antimycotic), the Cell Culture Insert gel was incubated for three days to ventralize the hindgut. The inducer medium b2 of 500 µL was added under the Cell Culture Insert gel, and the medium was exchanged every two days.

### Process c2: Transplantation under the renal capsule of immunocompromised mice

After ventralization of the hindgut (on day 14 after the induction of differentiation), the Cell Culture Insert gel was cut from the membrane of the Cell Culture Insert Transparent PET Membrane and placed on a 35mm dish. Then, ventral hindgut organoids were removed from the Matrigel of 2.5 mg/mL with tweezers under a stereomicroscope.

The renal membrane of a 4-week-old male NOD SCID JAX mouse (Oriental Yeast Co., Ltd.) was cut open with a blade under isoflurane inhalation anesthesia (isoflurane 1.3-1.4%, 190-200 mL/min), and the ventral hindgut organoid was implanted between the renal membrane and renal parenchyma. The fixed grafts were frozen, and frozen sections were prepared. The frozen sections were immunofluorescently stained and H&E staining.

The transplanted ventral hindgut organoid became a cellular structure (bladder organoid) with a pouch-like structure having a lumen after four weeks of transplantation, like the living bladder. The bladder organoid contained cells expressing the bladder epithelial markers UPK2 (Fig. 5a), P63 (Fig. 5b), and KRT5 (Fig. 5c), which formed a similar structure to the layer structure of the matured bladder *in vivo* (Fig. 5g). Specifically, the bladder organoid had Krt5-basal cell-like cells co-expressing P63 and KRT5 localized at the outermost periphery of the pouch-like cellular structure (Fig. 5e). In addition, a few Intermediate cell-like cells co-expressing UPK2 and P63 existed in an inward direction relative to the region where the KRT5-basal cell-like cells were localized (Fig. 5f). Furthermore, Superficial cell-like cells, expressing UPK2 but neither expressing P63 nor KRT5, were localized in an inward direction to the region where the Intermediate cell-like cells were localized and to the region facing the lumen of the bladder organoid (Fig. 5e and f).

Examples 2 and 3 showed that multiple types of bladder epithelial cells could be induced and that the cellular structures had a layer structure composed of these multiple types of bladder epithelial cells. Examples 2 and 3 showed that bladder-like three-dimensional structures composed of multiple species of bladder epithelial cells were formed. The comparison of the *in vitro* induction system (Example 2) with the *in vivo* induction system (Example 3) for differentiating hindgut organoids suggests that an induction-differentiation system where ventral hindgut organoids can be cultured for a long-term or cultured in the presence of Mesenchyme like *in vivo* to have a pouch-like lumen and to be matured is preferable.

### [Example 4] Production of ventral hindgut organoids

Figure 6 is a flowchart diagram indicating the culture to produce bladder organoids. Figure 6 indicates Process A for inducing differentiation of human iPS cells into definitive endoderm cells, Process b3 for inducing differentiation of the definitive endoderm cells into ventral hindgut organoids, and Process c3 for inducing differentiation from the ventral hindgut organoids into bladder organoids by co-culturing with mesenchymal cells or mesenchymal stem cells. The day indicated at the top of Figure 6 is the number of days required for the exemplary induction of differentiation according to one embodiment. Example 4 carries out Process A and Process b3 to produce ventral hindgut organoids.

### Pre-culture of human iPS cells

Human iPS cells were pre-cultured in the same way as in Example 1, except for seeding the cells at 45,000 cells/cm² onto a 6-well plate.

### Process A: Induction of differentiation into definitive endoderm cells

Human iPS cells were induced and differentiated into definitive endoderm cells in the same way as in Example 1, except for changing the concentration of the additional agent CHIR99021 in the inducer medium A, STEMdiff APEL2 medium, from 1 µM to 1.25 µM.

### Process b3: Induction of ventral hindgut differentiation

After induction of embryonic endoderm differentiation (on day 3 after the induction of differentiation), the inducer medium A was exchanged for an inducer medium b3, STEMdiff APEL2 medium (supplemented with 10 ng/ml BMP4, 200 ng/ml FGF4, 4µM CHIR99021, 1µg/mL Heparin, 2% PFHM-II, and Antibiotic-Antimycotic), and the differentiation-induced definitive endoderm cells were cultured for three days.

On day 5 after the induction of differentiation, culturing began with shaking at 100 rpm. Then, on day 6 after the induction of differentiation, floating spheroids were collected in 35 mm dishes. Next, the remaining spheroids in the wells from which the floating spheroids were collected were floated by pipetting a medium over the spheroids and collected in the same 35-mm dish. The collected spheroids were kept on ice.

Matrigel Growth Factor Reduced (Corning) (protein concentration at 10mg/mL) was diluted to 50% concentration in an inducer medium b3, added to Cell Culture Insert Transparent PET Membrane 24 well 8.0µm pore-size (corning) at 50 µL/well, and incubated at 37°C for 30 min to gel. Then, the collected spheroids of 60-70 were added to 50% Matrigel solution of 50 µL and mixed. The mixture was added onto the top of the gelled 50% Matrigel and incubated at 37°C under 5% CO₂ for 30 min to gel and obtain a Cell Culture Insert gel. The inducer medium b3 was added onto the top of the Cell Culture Insert gel at 200 µL and under the gel at 300 µL, and the gel was incubated at 37°C under 5% CO₂ for eight days (until day 14 after the induction of differentiation). The medium was exchanged daily in 2D culturing and every two days in 3D culturing. The spheroids in the 50% Matrigel grew by the culture in the inducer medium b3 and formed rounded cellular structures having a lumen (ventral hindgut organoids).

The ventral hindgut organoids on day 11 and day 14 after the induction of differentiation were immunofluorescently stained. Examples 4 and 5 used the following antibodies for immunofluorescence staining.
Primary antibodies used for immunofluorescence staining were as follows: Mouse Anti-CDX2 (1:100, BioGenex, #MU392-UC), Mouse Anti-FOXA2 (1:100, Santa Cruz Biotechnology, #sc-101060), Rabbit Anti-GATA3 (1:100, Cell Signaling, #5852S), Goat Anti-GATA3 (1:100, R&D Systems, #AF2605), Mouse Anti-ISL1&2 (1:100, DSHB, #39.4D5), Goat Anti-SOX2 (1:100, R&D Systems, #AF2018), Rabbit Anti-SATB2 (1:100, CELL MARQUE, #384R-14), Rabbit Anti-HOXA13 (1:200, Abcam, #ab106503), Goat Anti-ZO1 (1:100, ThermoFisher, #PA5-19090), Rabbit Anti-pSmad1/5/8 (1:100, Cell signaling, #13820), Rat Anti-CK8 (1:200, DSHB, #TROMA-1), Mouse Anti-Uroplakin II (1:100, BIOCARE MEDICAL, #ACR3051C), Rabbit Anti-ΔNP63 (1:100, Cell signaling, #67825), Rabbit Anti-P63 (1:100, Abcam, #ab124762), Chicken Anti-Keratin 5 (1:300, Bio Legend, #905903), Mouse Anti-ECAD (1:200, BD Transduction Laboratries, #610181), Goat Anti-ECAD (1:300, R&D SYSTEMS, #AF648), Chicken Anti-Vimentin (1:300, NOVUS, #NB300-223), Rabbit Anti-αSMA (1:100, Cell signaling, #19245T).

Secondary antibodies used for immunofluorescence staining were as follows: Alexa Fluor488 Donkey Anti-Mouse IgG (1:400, Life Technologies, #A21202), Alexa Fluor568 Donkey Anti-Rabbit IgG (1:400, Life Technologies, #A10042), Alexa Fluor647 Donkey Anti-Goat IgG (1:400, Life Technologies, #A21447), Alexa Fluor647 Donkey Anti-Chicken IgY (1:400, Jackson ImmunoResearch, #703-606-155).

The hindgut organoids did not have lumenal structures after the induction of hindgut differentiation (on day 11 after the induction of differentiation) in Process b1 in Example 1 (Fig. 7a). The ventral hindgut organoids had lumenal structures on day 5 after induction of ventral hindgut differentiation (on day 11 after the induction of differentiation) in Process b3 in Example 4 (Fig. 7b). Both hindgut and ventral hindgut organoids expressed the epithelial markers KRT8 and ECAD (Figs. 7a1, a10, b1, and b10). The hindgut organoids weakly expressed the ventral hindgut marker GATA3 (Fig. 7a2), while the ventral hindgut organoids strongly expressed it (Fig. 7b2). The hindgut organoids expressed the dorsal hindgut markers SOX2, CDX2, and T (Figs. 7a3 and a5-a7) but not the ventral hindgut organoid (Figs. 7b3 and b5-b7). The hindgut organoids did not express the intestinal marker FOXA2 (Fig. 7a9), while the ventral hindgut organoids did (Fig. 7b9). Thus, the hindgut organoids before ventralization of the hindgut in Process b2 in Example 1 had no luminal structure and expressed dorsal hindgut markers (SOX2, CDX2, and T), while weakly expressed or not ventral hindgut marker (GATA3) and intestinal marker (FOXA2) (Fig. 7a). In contrast, the ventral hindgut organoids during induction of ventral hindgut differentiation in Process b3 in Example 4 had luminal structures and did not express the dorsal hindgut marker but expressed the ventral hindgut marker and intestinal marker (Fig. 7b).

On day 8 of induction of ventral hindgut differentiation in Process b3 in Example 4 (on day 14 after the induction of differentiation), the ventral hindgut organoids were immunofluorescently stained and observed under a microscope (Fig. 8). The ventral hindgut organoids also expressed the epithelial tissue marker ECAD (Fig. 8d3) and a tight junction marker ZO1 (Fig. 8c3). The ventral hindgut organoids expressed the early intestinal epithelial markers FOXA2 (Fig. 8b1) and CK8 (Fig. 8c1), and the ventral hindgut markers GATA3 (Fig. 8a3, b3, and e1), ISL1 (Fig. 8d1), SATB2 (Fig. 8c2), and ΔNP63 (Fig. 8b2). The ventral hindgut organoids also expressed a ventral Cloaca region marker Phospho-Smad1/5/8 (Fig. 8d2) and HOXA13, a marker of the posterior intestinal tract and Cloaca region (Fig. 8f2). On the other hand, the ventral hindgut organoids did almost not express the dorsal hindgut markers CDX2 (Figs. 8a1 and f1) and SOX2 (Figs. 8a2 and e2). In addition, the ventral hindgut organoids expressed neither the bladder epithelial progenitor cell UPK2 (Fig. 8e3) nor the bladder epithelial cell marker KRT5 (Fig. 8f3). These results suggest that ventral hindgut organoids having luminal structures are formed by culturing in the inducer medium b3c for eight days (on day 14 after the induction of differentiation). The results also suggest forming ventral hindgut/Cloaca-like cells before becoming bladder epithelial progenitor cells.

### [Example 5] In vitro production of bladder organoids

Example 5 carries out Processes A, b3, and c3 shown in Figure 6 to produce bladder organoids *in vitro.* In Process C of Example 5, ventral hindgut organoids formed in Process b3 are cultured *in vitro* with bladder mesenchymal cells originating from E12.5 mice (Process c3).

### Pre-culture of human iPS cells

Human iPS cells were pre-cultured in the same way as in Example 4.

### Process A: Induction of differentiation into definitive endoderm cells

Human iPS cells were induced and differentiated into definitive endoderm cells in the same way as in Example 4.

### Process b3: Induction of ventral hindgut differentiation

The definitive endoderm cells were induced and differentiated into ventral hindgut organoids in the same way as in Example 4.

### Process c3: Induction of bladder epithelial differentiation by co-culture

### Preparation of E12.5 mouse bladder mesenchymal cells

An ICR mouse was euthanized by spinal dislocation on the 12th day of pregnancy. The fetal sac containing the fetuses was removed from the ICR mouse and placed in ice-cold 10% FBS/PBS(-). The fetuses were removed from the fetal sac with tweezers, and the placentas were cut out from the fetuses. Next, the upper half of the fetus's body was cut off with tweezers to expose the bladder region, and a region from the bladder to the urethra was collected. The collected region including the bladder and urethral was placed in 200 µL of Dissociation Buffer (Dispase: Corning #354235, 1 mg/mL DNaseI: Sigma-Aldrich #11284932001) and incubated at 37°C for 1 minute. Next, the region was diluted twice with an ice-cold M2 medium of 3 mL and kept on ice for 10 minutes. Then, bladder mesenchymal cells only above the site of ureter insertion were harvested with tweezers and a glass needle and collected in a 1.5-mL tube. Next, to the tube, TrypLE Select (Thermo Fisher Scientific) warmed at 37°C was added at 200 µL. The test tube was incubated at 37°C for 3 minutes and pipetted to separate the tissue into single cells. DMEM (supplemented with 10% FBS, GultaMAX-I, and Antibiotic-Antimycotic) was added at 1 mL to the tube to stop the enzyme reaction. The tube was centrifuged at 300 rcf for 3 minutes, and the supernatant was removed. To the precipitate, STEM-CELLBAMKER GMP grade (ZENOQ #CB045) was added at 250 µL and resuspended. The cell suspension was transferred to a cryopreservation tube and stored at -80°C.

### Co-culturing with E12.5 mouse bladder mesenchymal cells

The ventral hindgut organoids were separated from the extracellular matrix (50% Matrigel) gel obtained in Process b3 by pipetting and placed on ice.

Cryopreserved E12.5 mouse bladder mesenchymal cells were thawed in a water bath at 37°C and suspended in STEMdiff APEL2 medium of 1 mL. The obtained cell suspension was centrifuged at 300 rcf for 3 minutes, and the supernatant was removed. To the precipitate, STEMdiff APEL2 medium was added at 200 µL and resuspended. The obtained cell suspension was measured for cell counting, and the cell suspension was seeded onto a PrimeSurface^{®} plate 96V (Sumitomo Bakelite Co. Ltd., #MS-9096V) at 25,000 cells/well. The plate was centrifuged at 200 rcf for 5 minutes.

The ventral hindgut organoids separated from the Matrigel were seeded at one per well of the plate and allowed to stand until they settled. Then, the cell suspension was added to each well at 25,000 cells/well, and the plate was centrifuged at 200 rcf for 5 minutes. The ventral hindgut organoids were incubated with the bladder mesenchymal cells at 37°C under 5% CO₂ for one day. Next, aggregates of the cells in combination were transferred from the wells of the plate to a container, and the container was placed on a 3D rotary suspension culture device, CellPet 3D-iP (JTEC Corporation). The aggregates of the cells in combination were cultured in STEMdiff APEL2 medium (STEMCELL Tecnologies) supplemented with 2% PFHM-II and Antibiotic-Antimycotic or STEMdiff APEL2 medium (STEMCELL Tecnologies) supplemented with 2% PFHM-II, 2% FBS, and Antibiotic-Antimycotic on the CellPet 3D-iP at 37°C under 5% CO₂ for 11 days to induce bladder organoids (on day 24 after the induction of differentiation).

Bladder organoids were observed microscopically in a bright field on day 7 of co-culture with ventral hindgut organoids and bladder mesenchymal cells in Process c3 (on day 20 after the induction of differentiation) (Fig. 9). A dual structure consisting of an epithelial structure and a luminal structure was observed on the bladder organoids (Figs. 9a-c). Even in cases where a double structure is not apparently observed in a bright field (Fig. 9d), fluorescence staining enables observation of the double structure.

Bladder organoids had a spherical shape and a luminal structure on day 11 after the co-culture in Process c3 (on day 24 after the induction of differentiation). The bladder organoid was immunofluorescently stained and observed under a microscope (Fig. 10). The bladder organoid comprised a lumen, a layer of cells expressing the epithelial cell marker ECAD, the ECAD-expressing cell layer facing the lumen (Fig. 10b1), and a layer of cells expressing a mesenchymal cell marker VIM, the cellular layer being in an outward direction relative to the ECAD-expressing cell layer (Figs. 10b3 and b5). The bladder organoid comprised a layer of DAPI-stained cells in an outward direction relative to the ECAD-expressing cell layer (Figure 10c1) and a layer of cells expressing a smooth muscle cells marker αSMA in an outward direction relative to the DAPI-stained cell layer (Figs. 10c2 and c4). These results indicate that the bladder organoids comprise a lumen; an ECAD-expressing epithelial cell layer facing the lumen; a VIM-expressing stromal-like cell layer in an outward direction relative to the epithelial cell layer; and an αSMA-expressing smooth muscle cell layer in an outward direction relative to the stromal-like cell layer.

The cells of the ECAD cell layer in the bladder organoid co-expressed human lamin B1 (hLNB) (Figs. 10b2 and b). This result indicates that the epithelial cells of the bladder organoid originate from human iPS cells. Therefore, the bladder organoid has a lumen; an epithelial cell layer derived from human iPS cells facing the lumen; a stromal-like cell layer derived from mouse bladder mesenchymal cells in an outward direction relative to the epithelial cell layer; and a smooth muscle cell layer derived from mouse mesenchymal cells in an outward direction relative to the stromal-like cell layer.

Contractile movements were observed in the bladder organoids. This suggests that the smooth muscle cell layer functions in the bladder organoid.

The epithelial cell layer in the bladder organoid had a layer of UPK2-expressing cells on the innermost side (Fig. 10a1) and a layer of ΔNP63-expressing cells in an outward direction relative to the UPK2-expressing cell layer (Figs. 10a2 and a5). The result indicates that the bladder organoid has a similar structure to the developing bladder epithelium. Cells expressing a bladder epithelial marker KRT5 were not observed in the epithelial cell layer of the bladder organoid (Fig. 10a3). KRT5-expressing basal cells are known to appear in the late stages of bladder development. These results suggest that the bladder organoids on day 24 after the induction of differentiation in Example 5 are in the developing stages of bladder development. The epithelial cell layer in the bladder organoid expressed the bladder epithelial cell marker GATA3 (Fig. 10d2) and the developing bladder epithelial cell marker FOXA2 (Fig. 10d1). These results also suggest that the bladder organoids on day 24 after the induction of differentiation in Example 5 are in the developing stages of bladder development.

Bladder organoids were immunofluorescently stained and observed under a microscope on day 29 after co-culturing in Process c3 (on day 42 after the induction of differentiation) (Fig. 11). The bladder organoid had a luminal structure. The epithelial cell layer of the bladder organoid (Figure 11a5 and b5) comprised a layer of cells expressing UPK1B (Fig. 11a1) and UPK2 (Fig. 11b1), the layer facing a lumen; a layer of cells expressing ΔNP63 (Figs. 11a2 and b2), the layer being in an outward direction relative to the above-described layer; and a layer of cells expressing KRT5 (Figs. 11a3 and b3), the layer being in an outward direction relative to the above-described layer. Since KRT5-expressing basal cells are known to appear in the late stage of bladder development, these results suggest that the bladder organoid on day 42 after the induction of differentiation in Example 5 is in the late stage of bladder development.

## Claims

1. A method for producing a ventral hindgut organoid, comprising
culturing a pluripotent stem cell with an inducer medium A containing activin A and GSK3β inhibitor to induce differentiation into definitive endoderm cells and
culturing the definitive endoderm cells with an inducer medium B containing fibroblast growth factor, GSK3β inhibitor, and optionally further containing bone morphogenetic protein and then culturing them in the presence of extracellular matrix with an inducer medium B containing fibroblast growth factor, GSK3β inhibitor, and optionally further containing bone morphogenetic protein, to form a ventral hindgut organoid.

2. The method according to claim 1, wherein
the forming of the ventral hindgut organoid comprises, after culturing the definitive endoderm cells with the inducer medium B, culturing them in the presence of extracellular matrix with the inducer medium B to form a hindgut organoid and culturing the hindgut organoid in the presence of extracellular matrix with the inducer medium B to form the ventral hindgut organoid,
the inducer medium B for forming hindgut organoid contains the fibroblast growth factor and the GSK3β inhibitor, and the inducer medium B for forming ventral hindgut organoid contains the fibroblast growth factor, the GSK3β inhibitor, and the bone morphogenetic protein.

3. The method according to claim 1, wherein the inducer medium B for forming ventral hindgut organoid contains the fibroblast growth factor, the GSK3β inhibitor, and the bone morphogenetic protein.

4. A ventral hindgut organoid produced by the method according to any one of claims 1-3.

5. A ventral hindgut organoid,
expressing a ventral hindgut marker P63,
expressing HOXA13 and CK8/KRT8, and
not substantially expressing a dorsal hindgut marker SOX2.

6. A ventral hindgut organoid having a lumen,
expressing at least one ventral hindgut marker selected from the group consisting of P63, ΔN63, GATA3, ISL1, and SATB2,
expressing at least one marker selected from the group consisting of Phospho-Smad1/5/8, HOXA13, FOXA2, CK8/KRT8, and ECAD, and
not substantially expressing at least one dorsal hindgut marker selected from the group consisting of SOX2, T, and CDX2.

7. A method for producing a bladder organoid, comprising
culturing a ventral hindgut organoid produced by the method according to any one of claims 1-3 or a ventral hindgut organoid according to any one of claims 4-6 in the presence of extracellular matrix with an inducer culture C containing retinoic acid, fibroblast growth factor, and bone morphogenetic protein;
introducing the ventral hindgut organoid into a kidney or bladder or its peripheral area of a human or a non-human mammal; or
culturing the ventral hindgut organoid in the presence of mesenchymal stem cells or mesenchymal cells.

8. The bladder organoid produced by the method according to claim 7.

9. A bladder organoid comprising
a first cell layer comprising cells that express UPK1B and/or UPK2 and do not substantially express P63; and
a second cell layer localized in an outward direction relative to the first cell layer, the second cell layer comprising cells that co-express P63 and UPK1B and/or UPK2;
wherein the bladder organoid may further comprise a third cell layer localized in an outward direction relative to the second cell layer, the third cell layer comprising cells that co-express P63 and KRT5.

10. The bladder organoid according to claim 9, comprising a lumen surrounded by the first cell layer.

11. The bladder organoid according to claim 9 or 10, comprising a fourth cell layer localized to an outward direction relative to the third cell layer, the fourth cell layer comprising stromal-like cells, and optionally further comprising a fifth cell layer localized to an outward direction relative to the fourth cell layer, the fifth cell layer comprising smooth muscle cells.

12. A method for producing a non-human mammal comprising a ventral hindgut organoid or a bladder organoid in a kidney or bladder thereof or its peripheral area, the method comprising introducing a ventral hindgut organoid produced by the method according to any one of claims 1-3, a ventral hindgut organoid according to any one of claims 4-6, a bladder organoid produced by the method according to claim 7, or a bladder organoid according to any one of claims 8-11 into a kidney or bladder or its peripheral area of a non-human mammal.

13. A non-human mammal comprising a ventral hindgut organoid produced by the method according to any one of claims 1-3, a ventral hindgut organoid according to any one of claims 4-6, a bladder organoid produced by the method according to claim 7, or a bladder organoid according to any one of claims 8-11 in a kidney or bladder thereof or its peripheral area.

14. A regenerative medicine composition for treating bladder injury or disease, comprising a ventral hindgut organoid produced by the method according to any one of claims 1-3, a ventral hindgut organoid according to any one of claims 4-6, a bladder organoid produced by the method according to claim 7, or a bladder organoid according to any one of claims 8-11.

15. A method for assessing drug responsiveness to a test substance, comprising
contacting the test substance with a ventral hindgut organoid produced by the method according to any one of claims 1-3, a ventral hindgut organoid according to any one of claims 4-6, a bladder organoid produced by the method according to claim 7, a bladder organoid according to any one of claims 8-11, a non-human mammal produced by the method according to claim 12, or a non-human mammal according to claim 13, and
measuring drug responsiveness to the test substance in the ventral hindgut organoid, the bladder organoid, or the non-human mammal.
